(19) Europäisches Patentamt
European Patent Office
Office européen des brevets

(11) EP 4 076 447 B1

(12) **EUROPEAN PATENT SPECIFICATION**

(45) Date of publication and mention of the grant of the patent:
**17.01.2024 Bulletin 2024/03**

(21) Application number: **20824230.5**

(22) Date of filing: **15.12.2020**

(51) International Patent Classification (IPC):
***A61K 31/42*** (2006.01) ***A61K 31/422*** (2006.01)
***A61K 45/06*** (2006.01) ***A61P 33/14*** (2006.01)

(52) Cooperative Patent Classification (CPC):
**A61K 31/42; A61K 31/422; A61K 45/06; A61P 33/14**

(86) International application number:
**PCT/EP2020/086102**

(87) International publication number:
**WO 2021/122515 (24.06.2021 Gazette 2021/25)**

(54) **PARASITE CONTROL IN RUMINANTS**

PARASITENBEKÄMPFUNG BEI WIEDERKÄUERN

LUTTE ANTIPARASITAIRE CHEZ LES RUMINANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2019 EP 19216378**

(43) Date of publication of application:
**26.10.2022 Bulletin 2022/43**

(73) Proprietor: **Intervet International B.V.**
**5831 AN Boxmeer (NL)**

(72) Inventors:
- **O'NEILL, Peter, Andrew**
  **Macquarie Park, NSW 2113 (AU)**
- **FISARA, Petr**
  **Macquarie Park, NSW 2113 (AU)**
- **FLOCHLAY-SIGOGNAULT, Annie**
  **Madison, New Jersey 07940 (US)**
- **ZOLLER, Hartmut**
  **55270 Schwabenheim (DE)**
- **HUYGHE, Bruno**
  **49071 Beaucouzé Cedex (FR)**

(74) Representative: **Intervet International B.V.**
**Wim de Körverstraat 35**
**5831 AN Boxmeer (NL)**

(56) References cited:
**WO-A1-2009/003075**
**WO-A1-2019/115492**
**WO-A1-2019/122324**
**WO-A1-2020/225143**
**WO-A1-2021/000865**
**WO-A2-2009/002809**
**WO-A2-2009/024541**
**US-A1- 2017 239 218**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 076 447 B1

## Description

FIELD OF THE INVENTION

The present invention relates to the treatment or prevention of parasitic arthropod infestations of animals.

BACKGROUND OF THE INVENTION

Throughout the world, louse infestation of ruminants is an important problem that impairs the growth and performance parameters among sheep, goats, cattle- beef, dairy stocks and buffalos.

Such lice infesting ruminants are typically categorized into those that feed directly on blood of the host animal - sucking lice and those that feed on dead skin, secretions and bacteria normally found at the surface of the skin - biting or chewing lice.

Three species of sucking lice are known on sheep - *Linognathus pedalis, L. africanus* and L. *ovillus.* Sucking lice of cattle e.g. *Linognathus vituli, Haematopinus eurysternus* and *Solenopotes capillatus* feed on blood while biting/ chewing lice (*Bovicola bovis*) feed on dead skin cells, hair and oil secretions.

The biting or chewing louse- the Sheep body louse, *Bovicola ovis,* is the most prevalent and economically important louse species infesting sheep in all major sheep producing countries including Australia and New Zealand.

Sheep body lice are obligate parasites and complete their whole life cycle on the sheep. They feed on dead skin, secretions and bacteria normally found at the surface of the skin and move within the fleece of sheep depending on the temperature.

*Bovicola bovis* parasitize cattle of any age and size. *B. bovis* is found in temperate climates worldwide. It is most abundant in North America because of the numbers of cattle present. The louse is also mostly found in the winter and early spring because that is when the hair of the host animals is longest, and the cattle have not started shedding yet.

The main source of chewing lice infestation is from contact with lice infested animals and infestations are spread via direct contact, such as when animals are confined.

The size of the lice population on individual animals fluctuates during the year according to the season. In sheep the lice population is smallest immediately after summer shearing and peaks in mid-winter.

Shearing of sheep significantly reduces the size of the lice population by physical removal and subsequent exposure to environmental conditions. Strong ultraviolet light and very high temperatures reduce lice populations.

Lice cause irritation inducing rubbing, which results in damaged wool, and poor wool growth in sheep, as well as hide damage, discomfort and annoyance to the infested animal.

Lice Infestations can cause significant production losses in sheep in two ways:

1. Fleece damage: Lice cause intense irritation. Infested sheep bite themselves or rub against trees and fences, leading to fleece derangement, cotting or broken fibres. Lousy wool often has a yellow color and a distinctive smell caused by skin secretions, causing further downgrading.

2. Hide damage: Lice can trigger an immune response known as cockle, which causes thickening of the skin and pelt damage.

Apart from the direct economic costs, the continued use of conventional treatments to control lice infestation can have detrimental effects through residues from such treatment (especially residual dip or other fluids containing pesticide residues) entering the environment and food chain as well as on farmer safety.

Conventional methods of lice control include chemicals that are applied topically to the skin. For sheep the common methods are dips, jetting fluids, sprays and pour-on’s, which kill lice by contact.

All sheep body lice treatments currently registered (2019) in the major markets Australia and New Zealand are topically applied treatments with the active ingredients from the following chemical classes: Neonicotinoid (Imidacloprid, Thiacloprid), Spinosyn (Spinosad), Organophosphate (OP: temephos, diazinon), Macrocyclic lactone (ML: abamectin, ivermectin), Magnesium fluorosilicate/sulphur (MgFSi, sulphur, rotenone), Insect growth regulator (IGR: diflubenzuron, triflumuron), and Synthetic pyrethroid (SP: cypermethin, alphacypermethrin, deltamethrin).

Such products for sheep are applied by the following conventional topical methods.

Backline applications: Relatively small volumes of product are applied along the backs of the sheep from head to rump. From here, the chemical spreads over the surface of the body, assisted by contact between sheep.

Immersion (plunge and cage) dipping: In this method of treatment, sheep are completely immersed in dipping solution. Effective lice control relies on sheep being wet to skin level over their entire body; this can be very difficult because of the waterproofing effect of wool.

Shower dipping: Shower dipping relies on applying a high volume of dip wash from spray nozzles on a slowly

rotating boom to completely wet the sheep.

Hand-jetting: Hand-jetting is one method used for applying a lice treatment in long wool (greater than 6 weeks wool growth). Hand-jetting requires high pressures at the hand-piece for the liquid to penetrate the wool.

However, lice problems often reoccur in affected sheep flocks after treatment because small populations of surviving lice start to reproduce and farmers may notice sheep scratching, biting, pulled wool, or an infestation at shearing time.

Hence, because of the limited efficacy of the conventional topical administration methods frequent re-treatment is required that requires additional handling of the animals, increases the use of pesticides and harms the environment.

Therefore, an easy to apply lice treatment that effectively controls or eliminates existing lice infestation and protects from re-infestation would be very desirable in the sheep industry. International patent applications WO 2013/184006 and WO 2014/051440 of Bayer suggest to internally/ systemically administer spinosyn compounds (Spinosad) or neonicotinoid compounds (thiacloprid) for the control of chewing lice.

WO 2019/115492 discloses isoxazoline compounds, such as fluralaner for use in the treatment or prevention of parasites in live stock after a subcutaneous administration of an implant comprising said isoxazoline compound.

However, no long- term effective treatment of chewing lice infestation has been shown in the documents even under laboratory conditions and multiple high doses need to be administered over time to show a sufficient treatment effect and no protective effect against new lice infestation was demonstrated.

Up to now no product other than for topical administration for the control of biting/ chewing lice has been introduced in any market. Therefore, none of the prior art methods satisfies the needs of the sheep and cattle producer.

In cattle animals, similar applications and products are used (except cage dips that are not used). The most common application form is a pour-on topical administration.

Thus, there is a need in this art to find a better solution to the problem of controlling chewing lice infestations in the sheep and cattle industry, while not harming animals, the environment or humans.

## SUMMARY OF THE INVENTION

The invention provides a composition to control chewing/ biting lice of sheep independent of its wool length

Accordingly, in one embodiment the present invention relates to an isoxazoline compound of Formula (I)

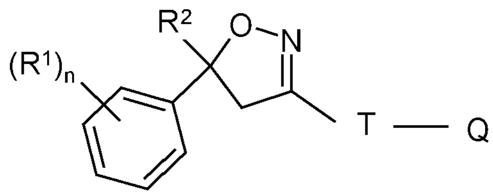

Formula (I),

wherein

$R^1$ is halogen, $CF_3$, $OCF_3$, CN,
n is an integer from 0 up to and including 3, preferably 1, 2 or 3,
$R^2$ is $C_1$-$C_3$-haloalkyl, preferably $CF_3$ or $CF_2Cl$,
T is a 5 to 12 membered mono or bicyclic ring system, which is optionally substituted by one or more radicals Y,
Y is methyl, halomethyl, halogen, CN, $NO_2$, $NH_2$-C=S, or two adjacent radicals Y form together a chain, especially a three or four-membered chain;
Q is X-$NR^3R^4$, $NR^5$-$NR^6$-X-$R^3$, X-R3 ora 5-membered N-heteroaryl ring, which is optionally substituted by one or more radicals;
X is $CH_2$, $CH(CH_3)$, CH(CN), CO, CS,
$R^3$ is hydrogen, methyl, haloethyl, halopropyl, halobutyl, methoxymethyl, methoxyethyl, halomethoxymethyl, ethoxymethyl, haloethoxymethyl, propoxymethyl,
ethylaminocarbonylmethyl, ethylaminocarbonylethyl, dimethoxyethyl, propynylaminocarbonylmethyl, N-phenyl-N-methyl-amino, haloethylaminocarbonylmethyl, haloethylaminocarbonylethyl, tetrahydrofuryl, methylaminocarbonylmethyl, (N,N-dimethylamino)-carbonylmethyl, propylaminocarbonylmethyl, cyclopropylaminocarbonylmethyl, propenylaminocarbonylmethyl, halo-ethylaminocarbonylcyclopropyl, alkylsulfanyl, alkylsufinalkyl, alkylsulfonalkyl, cycloalkyl

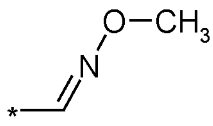 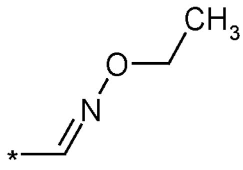 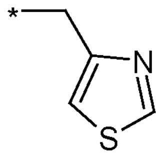

$R^3$-1 $R^3$-2 $R^3$-3

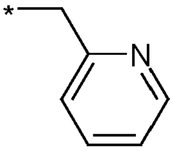 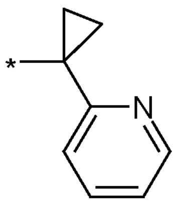 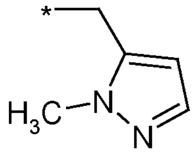 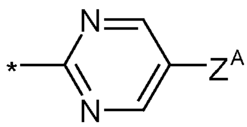

i. $R^3$-4 $R^3$-5 $R^3$-6 $R^3$-7

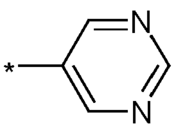 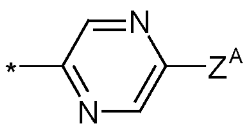 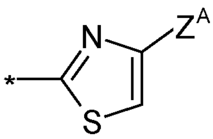

ii. $R^3$-8 $R^3$-9 $R^3$-10

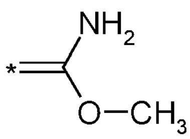 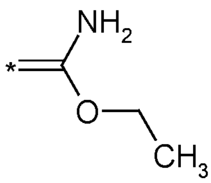 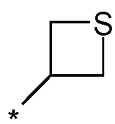 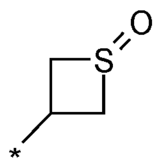

$R^3$-11 $R^3$-12 $R^3$-13 $R^3$-14

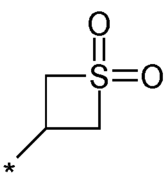 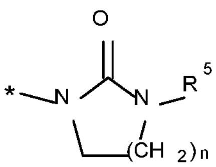 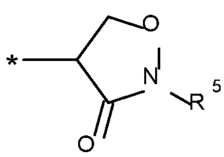 or 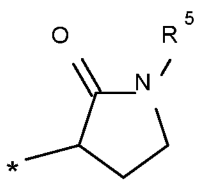

iii. $R^3$-15 $R^3$-16 $R^3$-17 $R^3$-18

wherein $Z^A$ is hydrogen, halogen, cyano, halomethyl, preferably $CF_3$;
$R^4$ is hydrogen, ethyl, methoxymethyl, halomethoxymethyl, ethoxymethyl, haloethoxymethyl, propoxymethyl, methylcarbonyl, ethylcarbonyl, propylcarbonyl, cyclopropylcarbonyl, methoxycarbonyl, methoxymethylcarbonyl, aminocarbonyl, ethylaminocarbonylmethyl, ethylaminocarbonylethyl, dimethoxyethyl, propynylaminocarbonylmethyl, haloethylaminocarbonylmethyl, cyanomethylaminocarbonylmethyl, or haloethylaminocarbonylethyl;

$R^5$ is hydrogen, alkyl or haloalkyl;
$R^6$ is hydrogen, alkyl or haloalkyl;

or $R^3$ and $R^4$ together form a substituent selected from the group consisting of:

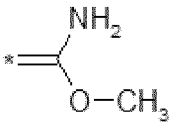, 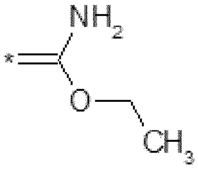, 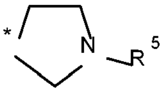, 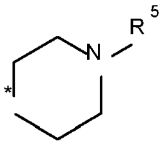, and 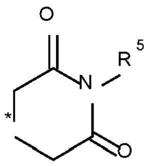

or a salt or solvate thereof for use in the treatment or protection of sheep from *Bovicola ovis* chewing lice infestation after a single subcutaneous administration of a composition comprising an effective amount of at least one isoxazoline compound of Formula (I), wherein the composition is administered as solution or suspension to sheep once > 6 weeks after shearing.

In one embodiment the invention provides a composition comprising an effective amount of such compound of Formula (I) or a salt or solvate thereof, when used for the elimination of chewing lice parasite infestations of sheep and/ or for the protection of sheep from infestation by chewing lice, wherein the composition is administered subcutaneously to sheep of any wool length.

In a preferred embodiment the isoxazoline compound of formula (I) is fluralaner.

The composition is administered once more than 6 weeks after shearing.

Another embodiment is a compound for use in a method of treating sheep that have been diagnosed or are suspected to be infested by chewing lice parasites comprising administering an effective amount of at least one isoxazoline compound of Formula (I) as defined in claim 1 or a salt or solvate thereof to the animals using a single subcutaneous administration.

Another embodiment is a compound for use in a method of protecting sheep from chewing lice infestation comprising administering an effective amount of an isoxazoline compound of Formula (I) as defined in claim 1 or a salt or solvate thereof to the animals using a single subcutaneous administration.

DETAILED DESCRIPTION OF THE INVENTION

It is known that lice infestations can only be successfully and sustainably treated in off-shears and short wool sheep, but not in long wool sheep. Existing backline products with long wool treatment claims are available as "emergency treatment" only because they are known to only reduce the lice burden and therefore require a re-treatment of the animal at the next shearing with a different product.

Other prior art methods for topical treatment are impractical in long wool sheep (such as immersion and shower dipping) because the long wool prevents effective wetting of the sheep. Therefore, there is a high desire for an effective product that can be used in sheep of all wool lengths, including in long wool.

For cattle animals it is known that only topical administration resulted in desirable control of chewing lice. However, such topical administration is not always desirable, especially for cattle animals that are exposed to outside weather with rain that can limit the effectiveness of topical products. Additionally, the subcutaneous injection delivers an exact dose, whereas for the pour-on administration there might be active lost due to run-off or licking by the same animal or other members of the cattle herd.

Isoxazoline compounds of Formula (I) are known to be effective to control blood sucking parasites such as fleas and ticks and that long- term efficacy and blood levels can be obtained in treated animal when using subcutaneous administration of such compounds.

It was very surprising that with a single subcutaneous administration of an effective amount of an isoxazoline compound as described in this application effective treatment and protection against non- blood sucking lice in sheep can be obtained.

As it has been shown in the examples, a single administration of a composition comprising an effective amount of isoxazoline compounds of formula (I) as described below, especially fluralaner, by subcutaneous administration to sheep can effectively treat existing lice infestations independent of the wool length, in long wool sheep, short wool sheep and off-shears sheep.

Additionally, one treatment provides protection of sheep against chewing lice re-infestation as shown in Example 5 in which treated sheep are challenged with chewing lice within 4 weeks after administration and no lice infestation is established on the treated sheep for more than 12 weeks.

The benefits of such method are that:

a) such method is more convenient than prior art application on animals because it avoids labor intensive techniques and avoids stress in the sheep animals when using such prior art topical methods;

b) Furthermore, the prior art application of parasiticides raises concerns in connection to animal safety and user safety during their administration, sheep can be handled immediately after treatment without exposure to the parasiticide; and

c) by this method an administration to a sheep independent of wool length during a defined time period is possible and therefore an effective control of the chewing lice population in a whole sheep flock and production unit even if sequential shearing is used.

d) the resistance breaking properties of such isoxazoline compounds are very favorable i.e. the chewing lice are very susceptible to the inhibiting or killing effect of such isoxazoline compounds

e) Generally, only one administration is required to effectively control / eliminate existing chewing lice infestation and no re-treatment of long wool sheep at next shearing would be required.

Consequently, by using the isoxazoline compounds as described in this application disadvantages of the prior art can be avoided, because a single convenient, safer administration of the isoxazoline compound would be sufficient to achieve the desired effect.

Hence the current invention would be a breakthrough in the control of sheep chewing/ biting lice, allowing lice effective control/ elimination of lice from sheep flocks independent of the wool length of the animals.

Definitions

The following definitions are relevant in connection with the embodiments of the present invention.

The present disclosure is particularly useful for ruminants such as cattle, sheep, goat, camels, llamas, especially when they are livestock animals, kept for meat and milk or wool or alternatively as working animals. In one non-claimed aspect the animals are bovine animals such as cattle. In this specification, bovine animals are mammals of the genus Bos and include, but are not limited to, cattle, steers, heifers, cows (lactating and non-lactating), calves, bulls, and also buffalo.

Preferred (non-claimed) are beef cattle, i.e. cattle animals kept for meat. In another non-claimed aspect the ruminants are dairy cattle, i.e. cattle animals kept for milk. According to the present invention, the ruminants are sheep.

*"Bovicola spp."* means chewing lice species parasitizing on ruminant animals such as *Bovicola ovis* or, *Bovicola bovis*

By "treating" or "treat" or "treatment" is intended the application or administration of an isoxazoline compound or composition to a ruminant, such as sheep or cattle animal that has been diagnosed to have a parasitic chewing lice infestation for the eradication of the parasite infestation or the reduction of the number of parasites, infesting the animal. It is a medical care given to animals infested with ectoparasites (WAAVP guidelines 2006)

The effect can be e.g. ovicidal, larvicidal and/or adulticidal or a combination thereof. The effect can manifest itself directly, i.e. killing the parasites either immediately or after some time has elapsed, for example when molting occurs, or by destroying their eggs, or indirectly, e.g. reducing the number of eggs laid and/or the hatching rate.

"Protection or protect " means that a new infestation of the ruminant animal, such as sheep or cattle, a sheep mob or flock or cattle herd with chewing lice parasites is prevented by killing adult parasites and any development/larval stages, that are able to infest the host, before infestation of the host or, by killing or inhibiting the parasites when they infest an animal that has been treated with an isoxazoline compound as described before or preventing generation of offspring of the parasites e.g. reducing the number of eggs laid and/or the hatching rate.

Protective periods may be e.g. useful when treated ewes contact untreated lambs, or when treated lambs contact untreated ewes infested with lice or when treated and untreated sheep become mixed. The same applies to cow-calf operations.

"Protection period" means the time, expressed in days or weeks after the treatment, that a veterinary test product will prevent the ectoparasite re-infestation of the animal hosts. Sometimes referred to as the prophylactic period or the persistent efficacy period (WAAVP guidelines 2006)

As used herein, an "active pharmaceutical ingredient "(or active ingredient, or pharmaceutically active ingredient or pharmaceutically acceptable active ingredient) is a substance used in a pharmaceutical product, intended to furnish pharmacological activity or to otherwise have direct effect in the diagnosis, cure, mitigation, treatment or prevention of disease, or to have direct effect in restoring, correcting or modifying physiological functions in humans or animals.

" Subcutaneous administration" is a method of administering a veterinary composition to an animal under the skin. In this type of injection, a needle is used to inject a liquid or solid (implant) drug into the tissue layer between the skin and the muscle. Conveniently the subcutaneous administration can be placed at the site of a body that is removed from the carcass for human consumption after slaughter. One such injection site for subcutaneous injection is the base of the ear / at the injunction of a pinna with the cranium of an animal, or other non-edible tissues such as the ear.

"Single administration" means that only one dosage of the isoxazoline compound is administered to a ruminant

such as sheep or cattle.

An "effective amount," is the amount or quantity of an isoxazoline compound as described above that is required to treat or prevent parasitic chewing lice infestations of animals, i.e. to alleviate or reduce lice numbers on an animal, and/or to inhibit the development of parasite infestations on an animal.

This amount is readily determined by observation or detection of the parasite numbers or eggs on the animal both before and after administering an isoxazoline compound as described above to such animals, e.g. the parasite count is reduced, after a first administration, by 5% to about 100%, preferably more than 50%, more than 70%, more than 90%, more than 95%, more than 99.5 % or 100%. Preferably the lice count is reduced by 100%, i.e. the effective amount results in elimination of all lice on a treated animal, i.e. that no chewing lice are found when the sheep are investigated as described above.

Depending on the specific isoxazoline compound used, the administration allows to completely inhibit or kill the chewing lice and preferably all viable eggs present on the animal.

In one embodiment the subcutaneous administration of the isoxazoline compound as described above results in an elimination/ eradication of chewing lice infestation. According to regulatory (Australian Pesticides and Veterinary Medicines Authority (APVMA)) guidelines in Australia 'Eradication' is defined as: 'Elimination of all live lice and viable eggs from treated animals, as determined by inspection of sheep 52 weeks after treatment.'

In current APVMA regulations the claim is applicable only to products applied off-shears or in short wool. Factors affecting the effective amount may include, for example, the parasite species to be treated and the development stages of the parasites, the wool length (see more details below) the type (e.g. species and breed, fine wool, coarse wool or hair wool), age, and condition of the infested animals; the environmental conditions (temperature, humidity), pharmacological considerations, such as the activity, efficacy, pharmacokinetic, and toxicology profiles of the particular isoxazoline compound administered; and whether the isoxazoline compound being administered is part of a combination of active ingredients. Thus, the preferred amount of the compound according to this invention can vary.

A lice infestation is diagnosed by inspecting the fleece of sheep especially the sheep suspected to be infested by lice with the naked eye. Lice can be found on most woolled areas of sheep. Densities of lice are highest along the sides and sometimes on the back of sheep. After shearing, a greater proportion of the population are found at sites on lower body regions such as under the neck, lower flanks and upper legs and in areas where the wool has not been closely shorn.

In cattle lice can be suspected when cattle show signs of rubbing. Rubbing causes hair loss which is commonly seen on the neck, shoulders and rump areas and is generally more severe than seasonal shedding of winter coat. If the coat is inspected thoroughly the lice infestation will be diagnosed.

For determining the level of infestation of lice in sheep and cattle , the WAAVP guidelines 2006 (Holdsworth, PA et al: "World Association for the Advancement of Veterinary Parasitology guidelines for evaluating the efficacy of ectoparasiticides against biting lice, sucking lice and sheep keds on ruminants ", Veterinary Parasitology 136 (2006), 45-54) specifically for sheep specify examination of 40 sites (partings), about 10 cm wide, in total per animal or 80 sites (partings), about 5 cm wide, per animal. The sites should be spaced so that they are representative of the full area of the body covered by the fleece on each side of the sheep.

In addition, the APVMA recommends that the examination sites are divided equally on each side of the animal, that is, 20 (10 cm) or 40 (5 cm) sites should be examined on each side of the body (total = 40 (10 cm) or 80 (5 cm) sites).

Furthermore, the APVMA recommends that the number of partings on each side of the body are distributed equally between the neck, shoulder, withers, rump and flank, that is, 4 (10 cm) or 8 (5 cm) partings at each location on each side of the body.

Preferably sheep are inspected for lice infestation and/ or existence of viable eggs 20 weeks after treatment.

In cattle lice should be according to the WAAVP counted on at least six sites per animal. These sites are determined during thorough examination of the animal prior to treatment (Day 1 or Day 0 louse count). At each site (about 5-15 cm) the hair should be parted at least three times to expose the skin and the total louse counts are recorded at each site.

The isoxazoline compounds for use in the current invention are known in the art and these compounds and their use as antiparasitics are described, for example, in US patent application US 2007/0066617, and International Patent applications WO 2005/085216, WO 2007/079162, WO 2009/002809, WO 2009/024541, WO 2009/003075, WO 2010/070068 and WO 2010/079077.

This class of compounds is known to possess excellent activity against blood sucking ectoparasites, i.e. parasitic insect and acarids, such as ticks and fleas.

In one embodiment the composition for use according to the invention comprises an isoxazoline compound of the Formula (I)

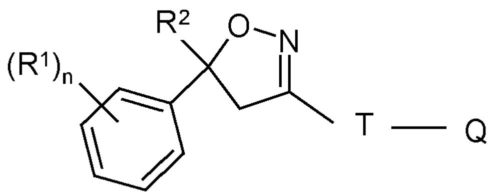

Formula (I), wherein
$R^1$ is halogen, $CF_3$, $OCF_3$, CN,
n is an integer from 0 up to and including 3, preferably 1, 2 or 3,
$R^2$ is $C_1$-$C_3$-haloalkyl, preferably $CF_3$ or $CF_2Cl$,
T is a 5 to 12 membered mono or bicyclic ring system, which is optionally substituted by one or more radicals Y,
Y is methyl, halomethyl, halogen, CN, $NO_2$, $NH_2$-C=S, or two adjacent radicals Y form together a chain, especially a three or four-membered chain;
Q is X-$NR^3R^4$, $NR^5$-$NR^6$-X-$R^3$, X-R3 ora 5-membered N-heteroaryl ring, which is optionally substituted by one or more radicals;
X is $CH_2$, $CH(CH_3)$, CH(CN), CO, CS,
$R^3$ is hydrogen, methyl, haloethyl, halopropyl, halobutyl, methoxymethyl, methoxyethyl, halomethoxymethyl, ethoxymethyl, haloethoxymethyl, propoxymethyl,
ethylaminocarbonylmethyl, ethylaminocarbonylethyl, dimethoxyethyl, propynylaminocarbonylmethyl, N-phenyl-N-methyl-amino, haloethylaminocarbonylmethyl, haloethylaminocarbonylethyl, tetrahydrofuryl, methylaminocarbonylmethyl, (N,N-dimethylamino)-carbonylmethyl, propylaminocarbonylmethyl, cyclopropylaminocarbonylmethyl, propenylaminocarbonylmethyl, halo-ethylaminocarbonylcyclopropyl, alkylsulfanyl, alkylsufinalkyl, alkylsulfonalkyl, cycloalkyl

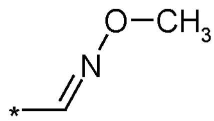

**$R^3$-1**

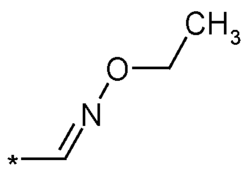

**$R^3$-2**

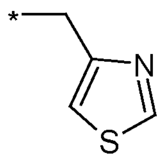

**$R^3$-3**

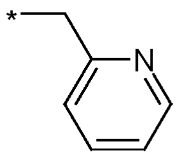

**$R^3$-4**

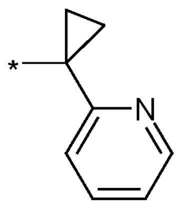

**$R^3$-5**

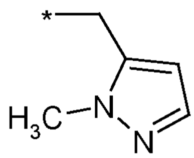

**$R^3$-6**

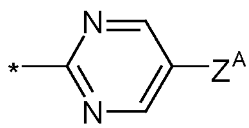

**$R^3$-7**

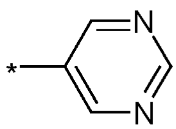

**$R^3$-8**

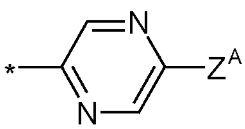

**$R^3$-9**

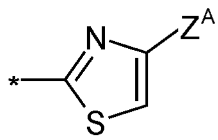

**$R^3$-10**

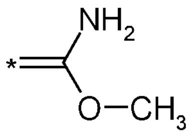

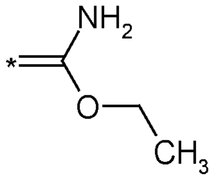

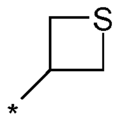

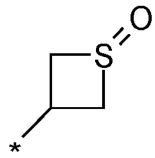

**$R^3$-11** **$R^3$-12** **$R^3$-13** **$R^3$-14**

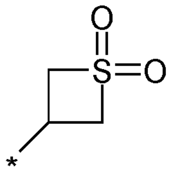

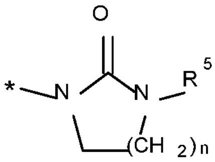

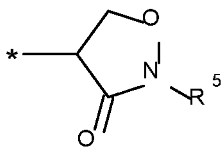

or

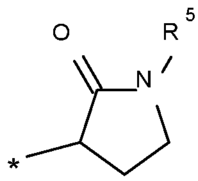

**$R^3$-15** **$R^3$-16** **$R^3$-17** **$R^3$-18**

wherein $Z^A$ is hydrogen, halogen, cyano, halomethyl, preferably $CF_3$;
$R^4$ is hydrogen, ethyl, methoxymethyl, halomethoxymethyl, ethoxymethyl, haloethoxymethyl, propoxymethyl, methylcarbonyl, ethylcarbonyl, propylcarbonyl, cyclopropylcarbonyl, methoxycarbonyl, methoxymethylcarbonyl, aminocarbonyl, ethylaminocarbonylmethyl, ethylaminocarbonylethyl, dimethoxyethyl, propynylaminocarbonylmethyl, haloethylaminocarbonylmethyl, cyanomethylaminocarbonylmethyl, or haloethylaminocarbonylethyl;
$R^5$ is hydrogen, alkyl or haloalkyl;
$R^6$ is hydrogen, alkyl or haloalkyl;
or $R^3$ and $R^4$ together form a substituent selected from the group consisting of:

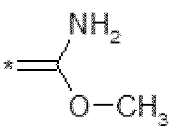,

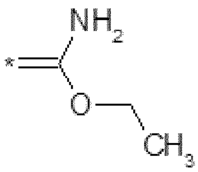,

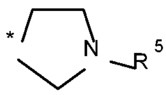,

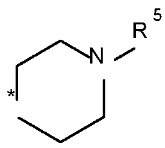

and

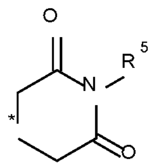

or a salt or solvate thereof.

In a preferred embodiment of the invention and/or embodiments thereof T is selected from

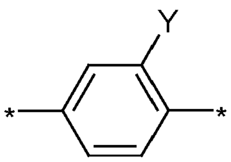

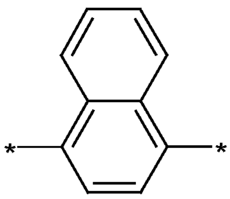

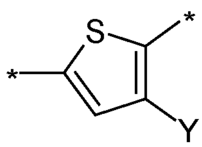

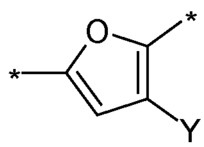

**T1** **T2** **T3** **T4**

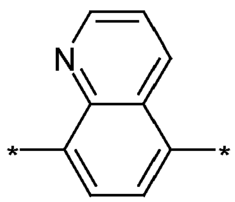

**T5**

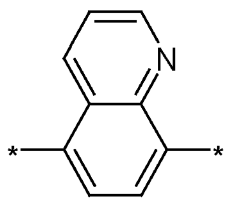

**T6**

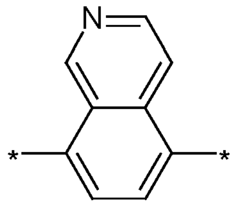

**T7**

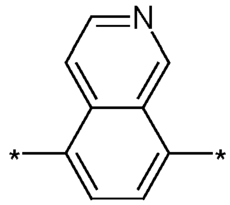

**T8**

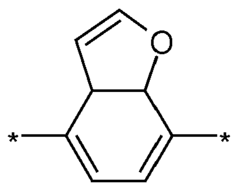

**T9**

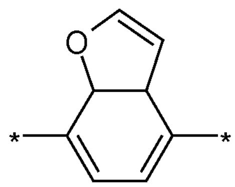

**T10**

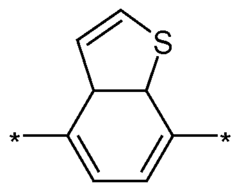

**T11**

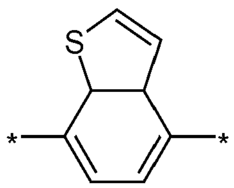

**T12**

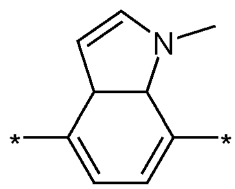

**T13**

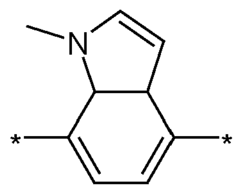

**T14**

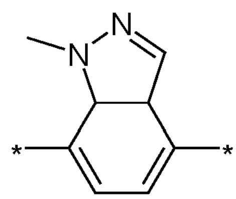

**15**

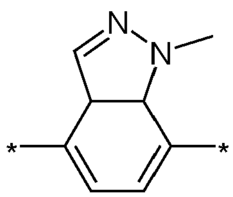

**T16**

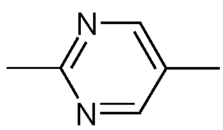

**17**

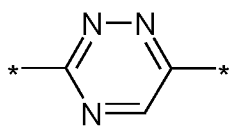

**T18**

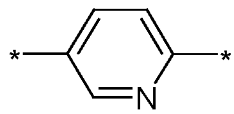

**T19**

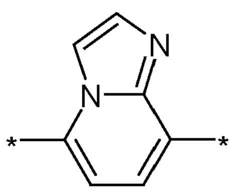

**T20**

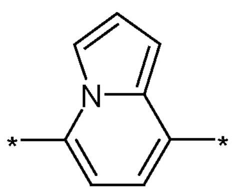

**T21**

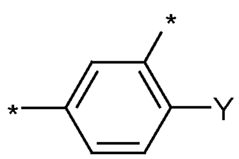

**T22**

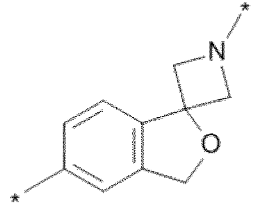

**T23**

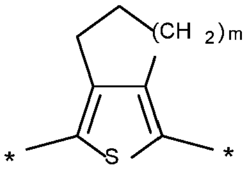 and 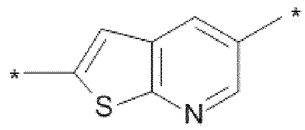

**T24** **T25**

wherein in T-1, T-3 and T-4, the radical Y is preferably hydrogen, halogen, methyl, halomethyl, ethyl or haloethyl.

In a preferred embodiment of the invention and/or embodiments thereof Q in Formula (I) is selected from

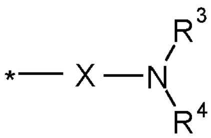 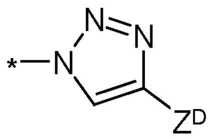 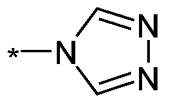 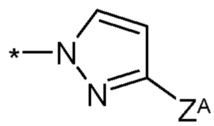

**Q-1** **Q-2** **Q-3** **Q-4**

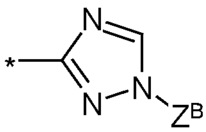 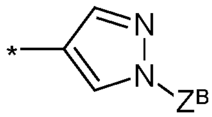 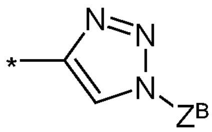 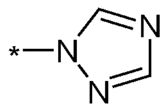 and

**Q-5** **Q-6** **Q-7** **Q-8**

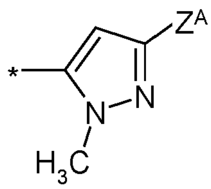

**Q-9**

wherein $R^3$, $R^4$, X and $Z^A$ are as defined above and $Z^B$ is

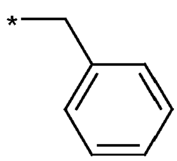 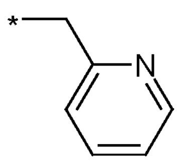 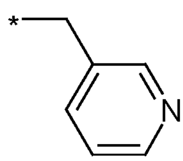 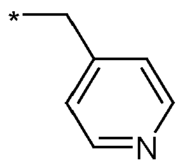

**$Z^B$-1** **$Z^B$-2** **$Z^B$-3** **$Z^B$-4**

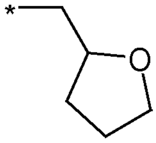
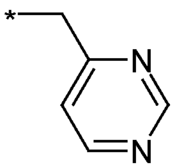
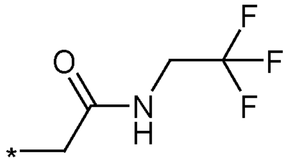
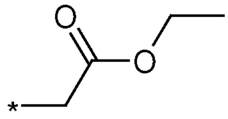

**Z^B-5** **Z^B-6** **Z^B-7** **Z^B-8**

or

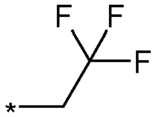

**Z^B-9**

$Z^D$ is

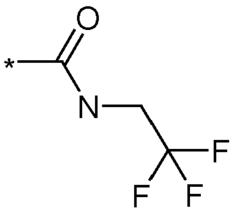
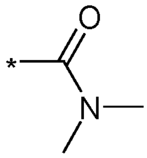
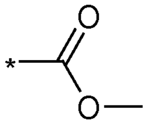
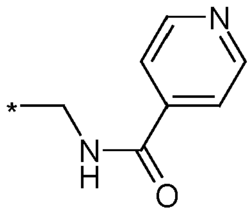
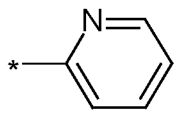

**Z^D-1** **Z^D-2** **Z^D-3** **Z^D-4** **Z^D-5**

or

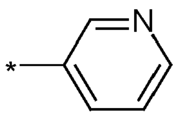

**Z^D-6**

Preferred compounds of Formula (I) are listed in Table 1:

Table 1:

| $(R^1)_n$ | $R^2$ | $R^3$ | $R^4$ | T | Y | Q | Z | X |
|---|---|---|---|---|---|---|---|---|
| 3-Cl, 5Cl | $CF_3$ | $CH_2CF_3$ | H | T-2 | - | Q-1 | - | C(O) |
| 3-Cl, 5Cl | $CF_3$ | $CH_2CH_3$ | H | T-2 | - | Q-1 | - | C(O) |
| 3-Cl, 5Cl | $CF_3$ | $CH_2CH_2OCH_3$ | H | T-2 | - | Q-1 | - | C(O) |
| 3-Cl, 5Cl | $CF_3$ | $CH_2C(O)NHCH_2CF_3$ | H | T-2 | - | Q-1 | - | C(O) |
| 3-Cl, 5Cl | $CF_3$ | $CH_2C(O)NHCH_2CH_3$ | H | T-2 | - | Q-1 | - | C(O) |
| 3-$CF_3$, 5-$CF_3$ | $CF_3$ | $CH_2C(O)NHCH_2CF_3$ | H | T-2 | - | Q-1 | - | C(O) |
| 3-$CF_3$, 5-$CF_3$ | $CF_3$ | $CH_2C(O)NHCH_2CH_3$ | H | T-2 | - | Q-1 | - | C(O) |
| 3-$CF_3$, 5-Cl | $CF_3$ | $CH_2C(O)NHCH_2CF_3$ | H | T-2 | - | Q-1 | - | C(O) |
| 3-$CF_3$, 5-Cl | $CF_3$ | $CH_2C(O)NHCH_2CH_3$ | H | T-2 | - | Q-1 | - | C(O) |

(continued)

| $(R^1)_n$ | $R^2$ | $R^3$ | $R^4$ | T | Y | Q | Z | X |
|---|---|---|---|---|---|---|---|---|
| 3-Cl, 5Cl | $CF_3$ | - | | T-2 | - | Q-6 | $Z^B$-7 | |
| 3-Cl, 5Cl | $CF_3$ | - | - | T-2 | - | Q-7 | $Z^B$-7 | |
| 3-Cl, 5Cl | $CF_3$ | - | - | T-2 | - | Q-5 | $Z^B$-7 | |
| 3-Cl, 5Cl | $CF_3$ | - | - | T-2 | - | Q-2 | $Z^D$-1 | |
| 3-Cl, 5Cl | $CF_3$ | $CH_2C(O)NHCH_2CF_3$ | H | T-3 | $CH_3$ | Q-1 | - | C(O) |
| 3-Cl, 5Cl | $CF_3$ | $CH_2C(O)NHCH_2CC$ | H | T-3 | $CH_3$ | Q-1 | - | C(O) |
| 3-Cl, 5Cl | $CF_3$ | $CH_2C(O)NHCH_2CN$ | H | T-3 | $CH_3$ | Q-1 | - | C(O) |
| 3-Cl, 5Cl | $CF_3$ | $CH_2C(O)NHCH_2CH_3$ | H | T-3 | $CH_3$ | Q-1 | - | C(O) |
| 3-$CF_3$, 5-$CF_3$ | $CF_3$ | $CH_2C(O)NHCH_2CF_3$ | H | T-3 | $CH_3$ | Q-1 | - | C(O) |
| 3-$CF_3$, 5-$CF_3$ | $CF_3$ | $CH_2C(O)NHCH_2CH_3$ | H | T-3 | $CH_3$ | Q-1 | - | C(O) |
| 3-Cl, 4-Cl, 5-Cl | $CF_3$ | $CH_2C(O)NHCH_2CF_3$ | H | T-3 | $CH_3$ | Q-1 | - | C(O) |
| 3-Cl, 4-Cl, 5-Cl | $CF_3$ | $CH_2C(O)NHCH_2CH_3$ | H | T-3 | $CH_3$ | Q-1 | - | C(O) |
| 3-Cl, 4-F, 5-Cl | $CF_3$ | $CH_2C(O)NHCH_2CF_3$ | H | T-3 | $CH_3$ | Q-1 | - | C(O) |
| 3-Cl, 4-F, 5-Cl | $CF_3$ | $CH_2C(O)NHCH_2CH_3$ | H | T-3 | $CH_3$ | Q-1 | - | C(O) |
| 3-Cl, 5-Cl | $CF_3$ | $CH_2C(O)NHCH_2CF_3$ | H | T-20 | - | Q-1 | - | C(O) |
| 3-Cl, 5-Cl | $CF_3$ | $CH_2C(O)NHCH_2CH_3$ | H | T-20 | - | Q-1 | - | C(O) |
| 3-$CF_3$, 5-$CF_3$ | $CF_3$ | $CH_2C(O)NHCH_2CF_3$ | $CH_3$ | T-20 | - | Q-1 | - | C(O) |
| 3-$CF_3$, 5-$CF_3$ | $CF_3$ | $CH_2C(O)NHCH_2CH_3$ | $CH_3$ | T-20 | - | Q-1 | - | C(O) |
| 3-$CF_3$, 5-$CF_3$ | $CF_3$ | $CH_2C(O)NHCH_2CF_3$ | H | T-20 | - | Q-1 | - | C(O) |
| 3-$CF_3$, 5-$CF_3$ | $CF_3$ | $CH_2C(O)NHCH_2CH_3$ | H | T-20 | - | Q-1 | - | C(O) |
| 3-$CF_3$, 5-$CF_3$ | $CF_3$ | $CH_2C(O)NHCH_2CF_3$ | H | T-21 | - | Q-1 | - | C(O) |
| 3-$CF_3$, 5-$CF_3$ | $CF_3$ | $CH_2C(O)NHCH_2CH_3$ | H | T-21 | - | Q-1 | - | C(O) |
| 3-Cl, 5-Cl | $CF_3$ | $CH_2C(O)NHCH_2CF_3$ | H | T-21 | - | Q-1 | - | C(O) |
| 3-Cl, 5-Cl | $CF_3$ | $CH_2C(O)NHCH_2CH_3$ | H | T-21 | - | Q-1 | - | C(O) |
| 3-Cl, 5-Cl | $CF_3$ | $CH_2CH_2SCH_3$ | H | T-21 | - | Q-1 | - | C(O) |
| 3-Cl, 4-Cl, 5-Cl | $CF_3$ | $C(O)CH_3$ | H | T-22 | F | Q-1 | - | $CH_2$ |
| 3-Cl, 4-Cl, 5-Cl | $CF_3$ | $C(O)CH(CH_3)_2$ | H | T-22 | F | Q-1 | - | $CH_2$ |
| 3-Cl, 4-Cl, 5-Cl | $CF_3$ | C(O)-cyclo-propyl | H | T-22 | F | Q-1 | - | $CH_2$ |
| 3-Cl, 4-F, 5-Cl | $CF_3$ | $C(O)CH_3$ | H | T-22 | F | Q-1 | - | $CH_2$ |
| 3-Cl, 4-Cl, 5-Cl | $CF_3$ | $C(O)CH_2CH_3$ | H | T-22 | F | Q-1 | - | $CH_2$ |
| 3-Cl, 4-F, 5-Cl | $CF_3$ | $C(O)CH_3$ | H | T-22 | Cl | Q-1 | - | $CH_2$ |
| 3-Cl, 5-Cl | $CF_3$ | $CH_2C(O)NHCH_2CF_3$ | H | T-1 | $CH_3$ | Q-1 | - | C(O) |
| 3-Cl, 5-Cl | $CF_3$ | $CH_2C(O)NHCH_2CH_3$ | H | T-1 | $CH_3$ | Q-1 | - | C(O) |
| 3-Cl, 5-Cl | $CF_3$ | $R^3$-1 (Z) | H | T-1 | $CH_3$ | Q-1 | - | C(O) |
| 3-Cl, 5-Cl | $CF_3$ | $R^3$-1 (E) | H | T-1 | $CH_3$ | Q-1 | - | C(O) |

More preferred compounds of Formula (I) are listed in Table 2.

Table 2:

| $(R^1)_n$ | $R^2$ | $R^3$ | $R^4$ | T | Y | Q | Z | X |
|---|---|---|---|---|---|---|---|---|
| 3-Cl, 5Cl | $CF_3$ | $CH_2CF_3$ | H | T-2 | - | Q-1 | - | C(O) |
| 3-Cl, 5Cl | $CF_3$ | $CH_2CH_3$ | H | T-2 | - | Q-1 | - | C(O) |
| 3-Cl, 5Cl | $CF_3$ | $CH_2CH_2OCH_3$ | H | T-2 | - | Q-1 | - | C(O) |
| 3-Cl, 5Cl | $CF_3$ | $CH_2C(O)NHCH_2CF_3$ | H | T-2 | - | Q-1 | - | C(O) |
| $3\text{-}CF_3$, $5\text{-}CF_3$ | $CF_3$ | $CH_2C(O)NHCH_2CF_3$ | H | T-2 | - | Q-1 | -- | C(O) |
| $3\text{-}CF_3$, 5-Cl | $CF_3$ | $CH_2C(O)NHCH_2CF_3$ | H | T-2 | - | Q-1 | | C(O) |
| 3-Cl, 5Cl | $CF_3$ | - | - | T-2 | - | Q-6 | $Z^B$-7 | |
| 3-Cl, 5Cl | $CF_3$ | - | - | T-2 | - | Q-7 | $Z^B$-7 | |
| 3-Cl, 5Cl | $CF_3$ | - | - | T-2 | - | Q-5 | $Z^B$-7 | |
| 3-Cl, 5Cl | $CF_3$ | - | - | T-2 | - | Q-2 | $Z^D$-1 | |
| 3-Cl, 5Cl | $CF_3$ | $CH_2C(O)NHCH_2CF_3$ | H | T-3 | $CH_3$ | Q-1 | - | C(O) |
| 3-Cl, 5Cl | $CF_3$ | $CH_2C(O)NHCH_2CC$ | H | T-3 | $CH_3$ | Q-1 | - | C(O) |
| 3-Cl, 5Cl | $CF_3$ | $CH_2C(O)NHCH_2CN$ | H | T-3 | $CH_3$ | Q-1 | - | C(O) |
| $3\text{-}CF_3$, $5\text{-}CF_3$ | $CF_3$ | $CH_2C(O)NHCH_2CF_3$ | H | T-3 | $CH_3$ | Q-1 | - | C(O) |
| 3-Cl, 4-Cl, 5-Cl | $CF_3$ | $CH_2C(O)NHCH_2CF_3$ | H | T-3 | $CH_3$ | Q-1 | - | C(O) |
| 3-Cl, 4-F, 5-Cl | $CF_3$ | $CH_2C(O)NHCH_2CF_3$ | H | T-3 | $CH_3$ | Q-1 | - | C(O) |
| 3-Cl, 5-Cl | $CF_3$ | $CH_2C(O)NHCH_2CF_3$ | H | T-20 | - | Q-1 | - | C(O) |
| $3\text{-}CF_3$, $5\text{-}CF_3$ | $CF_3$ | $CH_2C(O)NHCH_2CF_3$ | CH 3 | T-20 | - | Q-1 | - | C(O) |
| $3\text{-}CF_3$, $5\text{-}CF_3$ | $CF_3$ | $CH_2C(O)NHCH_2CF_3$ | H | T-20 | - | Q-1 | - | C(O) |
| $3\text{-}CF_3$, $5\text{-}CF_3$ | $CF_3$ | $CH_2C(O)NHCH_2CF_3$ | H | T-21 | - | Q-1 | - | C(O) |
| 3-Cl, 5-Cl | $CF_3$ | $CH_2C(O)NHCH_2CF_3$ | H | T-21 | - | Q-1 | - | C(O) |
| 3-Cl, 5-Cl | $CF_3$ | $CH_2CH_2SCH_3$ | H | T-21 | - | Q-1 | - | C(O) |
| 3-Cl, 4-Cl, 5-Cl | $CF_3$ | $C(O)CH_3$ | H | T-22 | F | Q-1 | - | $CH_2$ |
| 3-Cl, 4-Cl, 5-Cl | $CF_3$ | $C(O)CH(CH_3)_2$ | H | T-22 | F | Q-1 | - | $CH_2$ |
| 3-Cl, 4-Cl, 5-Cl | $CF_3$ | C(O)-cyclo-propyl | H | T-22 | F | Q-1 | - | $CH_2$ |
| 3-Cl, 4-F, 5-Cl | $CF_3$ | $C(O)CH_3$ | H | T-22 | F | Q-1 | - | $CH_2$ |
| 3-Cl, 4-Cl, 5-Cl | $CF_3$ | $C(O)CH_2CH_3$ | H | T-22 | F | Q-1 | - | $CH_2$ |
| 3-Cl, 4-F, 5-Cl | $CF_3$ | $C(O)CH_3$ | H | T-22 | Cl | Q-1 | - | $CH_2$ |
| 3-Cl, 5-Cl | $CF_3$ | $CH_2C(O)NHCH_2CF_3$ | H | T-1 | $CH_3$ | Q-1 | - | C(O) |
| 3-Cl, 5-Cl | $CF_3$ | $R^3$-1 (Z) | H | T-1 | $CH_3$ | Q-1 | - | C(O) |
| 3-Cl, 5-Cl | $CF_3$ | $R^3$-1 (E) | H | T-1 | $CH_3$ | Q-1 | - | C(O) |

In a particularly preferred embodiment of the invention and/or embodiments thereof the isoxazoline compound is represented by Formula (II)

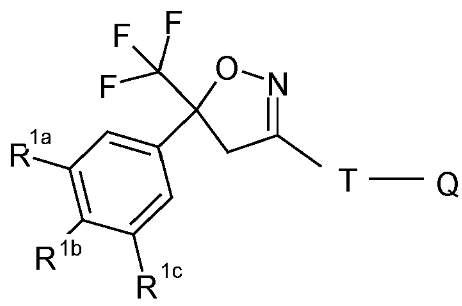

**Formula (II)**

wherein

$R^{1a}$, $R^{1b}$, $R^{1c}$ are independently from each other hydrogen, Cl or $CF_3$. Preferably $R^{1a}$ and $R^{1c}$ are Cl or $CF_3$ and $R^{1b}$ is hydrogen,

T is

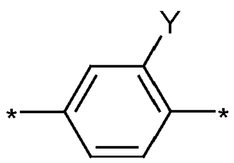

**T-1**

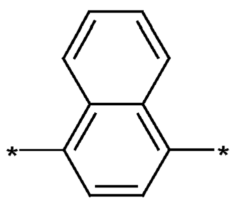

**T-2**

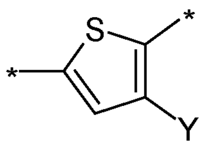

**T-3**

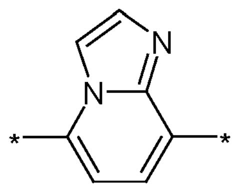

**T-20**

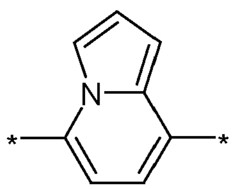

**T-21**

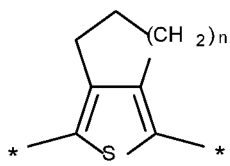

**T-23**

or

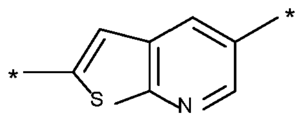

**T-24**

wherein Y is methyl, bromine, Cl, F, CN or $C(S)NH_2$ and

Q is as described above.

In another preferred embodiment of the invention and/or embodiments thereof $R^3$ is H and $R^4$ is $-CH_2-C(O)-NH-CH_2-CF_3$, $-CH_2-C(O)-NH-CH_2-CH_3$, $-CH_2-CH_2-CF_3$ or $-CH_2-CF_3$.

In another preferred embodiment of the invention and/or embodiments thereof the isoxazoline compound is selected from fluralaner, afoxolaner, sarolaner, lotilaner and tigolaner.

In one preferred embodiment of the invention and/or embodiments thereof the isoxazoline compound is 4-[5-(3,5-dichlorophenyl)-5-trifluoromethyl-4,5-dihydroisoxazol-3-yl]-2-methyl-*N*-[(2,2,2-trifluoro-ethylcarbamoyl)-methyl]-benzamide (CAS RN 864731-61-3). This compound is also known as fluralaner. In one preferred embodiment of the invention and/or embodiments thereof the isoxazoline compound is 4-[5-[3-chloro-5-(trifluoromethyl)phenyl]-4,5-dihydro-5-(trifluoromethyl)-3-isoxazolyl]-N-[2-oxo-2-[(2,2,2-trifluoroethyl)amino]ethyl]-1-naphthalenecarboxamide (CAS RN1093861-60-9). This compound is also known as a 4-[5-(5-chloro-α,α,α-trifluoro-*m*-tolyl)-4,5-dihydro-5-(trifluoromethyl)-1,2-oxazol-3yl]-*N*-[2-oxo-2-[(2,2,2-trifluoroethylamino]ethyl]naphthalene-1-or afoxolaner. Afoxolaner is for example disclosed in WO 2007/079162.

In one preferred embodiment of the invention and/or embodiments thereof the isoxazoline compound is

1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethan-1-one, preferably 1-(5'-((5*S*)-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethan-1-one (CAS RN: 1398609-39-6). This compound is known as sarolaner.

In one preferred embodiment of the invention and/or embodiments thereof the isoxazoline compound is 3-methyl-N-(2-oxo-2-((2,2,2-trifluoroethyl)amino)ethyl)-5-[5-(3,4,5-trichlorophenyl)-5-(trifluoromethyl)-4,5-dihydroisoxazol-3-yl]thiophene-2-carboxamide, preferably methyl-N-(2-oxo-2-((2,2,2-trifluoroethyl)amino)ethyl)-5-[(5S)-5(3,4,5-trichlorophenyl)-5-(trifluoromethyl)-4,5-dihydroisoxazol-3-yl]thiophene-2-carboxamide (CAS RN: 1369852-71-0). This compound is known as lotilaner.

In one preferred embodiment of the invention and/or embodiments thereof the isoxazoline compound is 2-chloro-*N*-(1-cyanocyclopropyl)-5-[1-[2-methyl-5-(1,1,2,2,2-pentafluoroethyl)-4-(trifluoromethyl)pyrazol-3-yl]pyrazol-4-yl]benzamide (CAS RN 1621436). This compound is known as tigolaner.

In one preferred embodiment of the invention and/or embodiments thereof the isoxazoline compound is (Z)-4-[5-(3,5-dichlorophenyl)-5-trifluoromethyl-4,5-dihydroisoxazol-3-yl]-N-[(methoxyimino)methyl]-2-methylbenzamide (CAS RN 928789-76-8).

In one preferred embodiment of the invention and/or embodiments thereof the isoxazoline compound is 4-[5-(3,5-dichlorophenyl)-5-(trifluoromethyl)-4H-isoxazol-3-yl]-2-methyl-N-(thietan-3-yl)benzamide (CAS RN 1164267-94-0) that was disclosed in WO 2009/0080250.

In one preferred embodiment of the invention and/or embodiments thereof the isoxazoline compound is 5-[5-(3,5-dDichlorophenyl)-4,5-dihydro-5-(trifluoromethyl)-3-isoxazolyl]-3-methyl-N-[2-oxo-2-[(2,2,2-trifluoroethyl)amino]ethyl]-2-thiophenecarboxamide (CAS RN 1231754-09-8) that was disclosed in WO 2010/070068.

Especially preferred is fluralaner (corresponding to 4-[5-(3,5-dichlorophenyl)-5-trifluoromethyl-4,5-dihydroisoxazol-3-yl]-2-methyl-*N*-[(2,2,2-trifluoro-ethylcarbamoyl)-methyl]-benzamide).

The invention comprises the use of racemic mixtures, for example, equal amounts of the enantiomers of such isoxazoline compounds as described above. In addition, the method of this invention includes isoxazoline compounds that are enriched compared to the racemic mixture in an enantiomer of Formula (I). Also included are the essentially pure enantiomers of such isoxazoline compounds.

When enantiomerically enriched, one enantiomer is present in greater amounts than the other, and the extent of enrichment can be defined by an expression of enantiomeric excess ("ee"), which is defined as (2x-l)-100 %, where x is the mole fraction of the dominant enantiomer in the mixture (e.g., an ee of 20 % corresponds to a 60:40 ratio of enantiomers). Preferably the compositions for use in the current invention have at least a 50 % enantiomeric excess; more preferably at least a 75 % enantiomeric excess; still more preferably at least a 90 % enantiomeric excess; and the most preferably at least a 94 % enantiomeric excess of the more active isomer. Of particular note are enantiomerically pure embodiments of the more active isomer.

Isoxazoline compounds as described above can comprise additional chiral centers. The method of this invention comprises racemic mixtures as well as enriched and essentially pure stereo configurations at these additional chiral centers.

For example, the isoxazolines comprise a chiral (or asymmetric) carbon at the 5-position of the isoxazoline ring. In some embodiments, for example, the chiral carbon has a left-handed (or "S" or "sinister") configuration. An example of such a compound is:

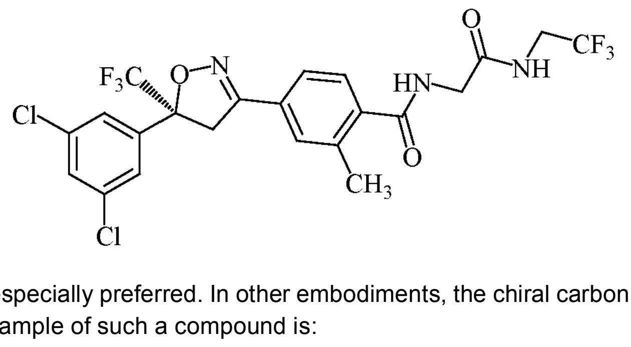

Such compound is especially preferred. In other embodiments, the chiral carbon has a right-handed (or "R" or "rectus") configuration. An example of such a compound is:

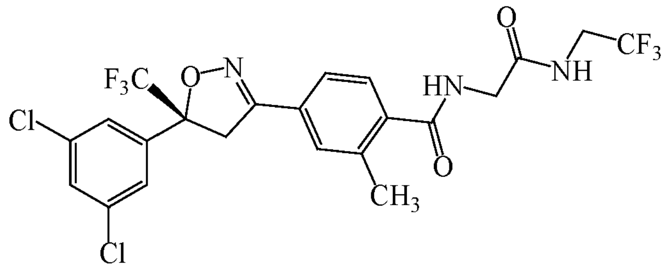

In one preferred embodiment the active enantiomer (s)-fluralaner is used.

In another preferred embodiment the active enantiomer (s)-afoxolaner is used.

Unless otherwise stated, an isoxazoline structure that does not indicate a particular conformation is intended to encompass compositions of all the possible conformational isomers of the isoxazoline, as well as compositions comprising fewer than all (e.g., just one of) the possible conformational isomers.

The reference to isoxazoline compound in this specification includes enantiomers, salts and solvates thereof that can be produced by conventional methods.

The term "salt" refers to salts prepared from pharmaceutically acceptable non-toxic bases or acids including inorganic or organic bases and inorganic or organic acids.

The term "solvate" is used herein to describe a molecular association comprising one or more pharmaceutically acceptable solvent molecules, e.g. water or ethanol. The term "hydrate" is used when said solvent is water.

Isoxazoline compounds of Formula (I) can be prepared according to one or other of the processes described e.g. in patent applications US 2007/0066617, WO 2007/079162,

WO 2009/002809, WO 2009/080250, WO 2010/070068, WO 2010/079077, WO 2011/075591 and WO 2011/124998 or any other process coming within the competence of a person skilled in the art who is an expert in chemical synthesis. For the chemical preparation of the products of the invention, a person skilled in the art is regarded as having at her/his disposal, inter alia, the entire contents of "Chemical Abstracts" and of the documents cited therein.

In one embodiment with the current invention existing chewing lice infestation can be controlled, preferably eliminated within 15 days after administration.

In one embodiment with the current invention viable chewing lice eggs from treated animals cannot be found after evaluation of the sheep within 42 days after administration In one embodiment with the current invention after a single administration no live lice and viable eggs can be found when investigating treated sheep 140 days after treatment or in cattle 56 days after treatment.

It has been also discovered that the current invention can be also used to control (treat and/ or prevent infestation) resistant lice populations of sheep.

Resistance can be defined as exposure to a low dose of pesticide that enables some lice to survive, and these have genes that may allow them to survive higher doses that would normally kill all lice. Continued use of the same chemical or chemical group allows the resistant lice to survive, breed and increase in numbers until they make up the majority of the population. Sometimes, when resistance is present, treatment suppresses lice, but does not completely eradicate them. These suppressed infestations are difficult to detect and increase the chance of lice spreading between flocks.

The current invention can be used on sheep that are infested with lice resistant to any one of organophosphates, synthetic pyrethroids, neonicotinoids, spinosyn or benzoyl phenyl urea compounds and will still provide effective treatment/ protection.

In one embodiment the current invention is able to protect sheep for at least 4 weeks from infestation by chewing lice

Lice infestations that can be treated/ prevented by the current invention can be present in various breeds of sheep including coarse wool sheep and fine wool sheep breeds such as Merino. It is known that sheep breeds have a different susceptibility to lice infestation. Merino breeds appear to be more susceptible than other breeds, but all breeds of sheep, including shedding breeds, such as Dorper and Damaras, can carry lice. The invention works in all such sheep breeds.

An important aspect of the current invention is that sheep with any length of wool can be successfully treated. This means an application within 24 hours after shearing- off shears, or during the time period >24 hours after shearing up to 6 weeks after shearing- short wool (not claimed) or > 6 weeks after shearing- long wool.

The current invention can be applied both on individual sheep that are lice infested or on sheep flocks with at least one sheep diagnosed with chewing lice infestation. Especially preferred and beneficial is the administration to sheep flocks.

It has been up to now easier to eradicate sheep lice on farms with one main shearing than it is on properties

where mobs are shorn at different times (split shearing). The current invention allows successful control of lice infestations even under such conditions which is a major advantage for the management of sheep farms.

In the past flock management became more complicated where pregnant ewes or ewes with lambs at foot needed to be treated for lice. This is because backline treatments and insect growth regulator products take some time to bring about the death of all lice. During this time, if there is contact with other untreated sheep, there is the potential for lice to spread.

Where split shearing occurs, it was in the past critical that treated sheep flocks are kept separate from untreated sheep until the untreated sheep are treated. Managing ewes with lambs at foot or ewes due to lamb soon after treatment is more complex. Especially in such situation it is very advantageous if a product protects sheep from re-infestation.

Typically effective (dosage) amount of isoxazoline compounds (especially fluralaner), are between 0.1 mg/kg bodyweight of the treated animal and 50 mg/kg bodyweight, or 0.25 mg/kg bodyweight to 10 mg/ kg bw, or 0.5 mg/ kg bw to 7.5 mg/kg bw , or 1 to 5 mg/kg bw . In one embodiment the effective dosage is 1.5 mg/kg bodyweight. Especially preferred, in case of fluralaner, is a dosage between about 1 and about 3 mg/ kg bodyweight of the animal that has been shown to eliminate lice infestation after single administration.

In one embodiment a single dose of an effective amount of the isoxazoline compound is administered to a sheep, or a flock of sheep, that has been diagnosed to be infested with chewing lice.

In one embodiment a single dose of an effective amount of the isoxazoline compound is administered to a sheep, or a flock of sheep, that has been in contact with sheep that has been diagnosed with a chewing lice infestation and is therefore at risk to be infested.

It has been surprisingly found that an extended duration of effective control of lice infestation is possible without the composition being an extended release composition to provide blood levels of the isoxazoline compound of formula (I) during the whole protection and/or treatment period. The term "extended release" or "extended release composition" as used herein means a dosage form that is formulated in such a manner to make a pharmaceutically active ingredient to be available over an extended period of time due to the interaction of the formulation components/ excipients in combination with the natural pharmacokinetic or pharmacodynamic characteristics of the active agent(s).

This definition is consistent with the use of the term known and accepted in the veterinary field as described in the article "Terminology Challenges: Defining Modified Release Dosage Forms in Veterinary Medicine" by Marilyn N. Martinez, Danielle Lindquist and Sanja Modric (Journal of Pharmaceutical Sciences, vol. 99, no. 8, August 2010).

A number of veterinary compositions are known to be suitable for subcutaneous injection administration to animals, generally liquid compositions such as solutions or suspensions of the isoxazoline compounds of Formula (I) using pharmaceutically acceptable solvents and suspending agents.

The pharmaceutical excipients are excipients with which the person skilled in the art is familiar, such as those which are described in the European Pharmacopoeia.

The composition for subcutaneous administration can be either a liquid solution or suspension. A solution is a mixture of two or more components that form a single phase that is homogeneous down to the molecular level. A suspension consists of insoluble solid particles dispersed in a liquid medium, with the solid particles accounting for about 0.5% to about 30% of the suspension. The liquid may be aqueous, oily, or both.

An example for a suitable injectable solution of isoxazoline compounds, especially fluralaner, is a pharmaceutical composition that comprises an isoxazoline compound, and a pharmaceutically acceptable carrier comprising 2 pyrrolidone, PEG and propylene glycol. An alternative is an aqueous suspension such as described in the examples.

## Combinations

This invention is also directed to composition for use according to the invention comprising more than one pharmaceutically active ingredient, i.e. wherein the composition for subcutaneous administration comprises another active pharmaceutical ingredient.

Those of ordinary skill in the veterinary pharmaceutical arts will be entirely familiar with the identity of such active ingredients which may include, without limitation antiparasitics such as endoparasitics or endoparasiticides (including anthelmintics) and endecto-parasticides, hormones and/or derivatives thereof, and minerals and vitamins.

Especially preferred is a combination with selenium, especially as sodium selenate.

Especially preferred are combinations with macrocyclic lactones such as ivermectin, moxidectin, abamectin, doramectin or eprinomectin.

In an alternative embodiment the composition includes a (fixed) combination of an effective amount of an isoxazoline compound for controlling chewing lice and another active ingredient that controls a different parasite infestation, e.g. against parasitic helminths or alternatively fly strike. In a specific embodiment two isoxazoline compounds as described above are included that control different parasites.

The amounts of each of the components in the final product may be varied considerably, depending upon the nature of the pharmaceutically active ingredients, the weight and condition of the subject treated, and the unit dosage

desired. Those of ordinary skill in the art will be able to adjust dosage amounts for particular pharmaceutically active ingredients in the composition in light of the teachings of this disclosure.

The product according to the current invention conventionally further comprises physiologically acceptable formulation excipients known in the art e.g. as described in "Gennaro, Remington: The Science and Practice of Pharmacy" (20th Edition, 2000).

All such ingredients, carriers and excipients must be substantially pharmaceutically or veterinary pure and non-toxic in the amounts employed and must be compatible with the pharmaceutically active ingredients.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

Preferred embodiments according to the invention are defined hereinafter. The preferred embodiments are preferred alone or in combination. Further, it is to be understood that the following preferred embodiments refer to all aspects of the present invention, i.e. the composition for use in a method of treating or protecting according to claim 1.

All reference in this document to "Lice" or "louse" is meant the parasitic sheep or cattle louse (chewing or biting louse), *Bovicola spp, especially B. ovis,* or *B. bovis* unless specifically indicated otherwise.

Examples Example 1

**Efficacy Long Wool Lice Study**

In this study the ability of subcutaneously administered fluralaner to control sheep body lice *(Bovicola ovis)* infestations in long wool sheep was evaluated.

Twenty-eight fine wool Merino ewes with 7 months wool growth and a heavy sheep body louse infestation (>5 lice per 10 cm parting) of benzoyl phenyl urea (IGR) resistant lice were divided into four groups of six animals such that each group had a similar mean lice burden. On Day 0 the animals in Groups 2 to 4 were weighed and treated with 1, 2 and 3 mg fluralaner/kg bodyweight respectively. The dose was administered by subcutaneous injection. Group 1 was left untreated as a negative control.

Lice counts were conducted pre-treatment (Day -1) and 7, 14, 21, 42, 84, and 140 days post-treatment. Lice counts were performed according to the World Association for the Advancement of Veterinary Parasitology (W.A.A.V.P.) "Guidelines for evaluating the efficacy of ectoparasiticides against biting lice, sucking lice and sheep keds on ruminants" and the APVMA Pre-amble to these guidelines. Twenty (20) sites on each side of the body were examined by opening the fleece about 10cm and counting all live lice seen. The total count from the forty (40) sites constituted the body count for each animal. The sites examined were spaced so that they were representative of the full area of the body covered by the fleece on each side of the sheep.

The percentage efficacy was calculated for each treatment group at each assessment time using the geometric mean lice counts and the Henderson-Tilton formula:

$$\text{Percentage Efficacy} = (1-(Ta/Ca) \times (Cb/Tb)) \times 100$$

Where:

- Ta = the mean number of lice on the treated group post treatment.
- Ca = the mean number of lice on the negative control group post treatment.
- Tb = the mean number of lice on the treated group before treatment.
- Cb = the mean number of lice on the negative control group before treatment.

The percentage reduction in lice count recorded in this study are presented in the table below. All dose levels of Fluralaner 50 mg/mL Injectable Solution provided 100% lice control from Day 21 (the 2.0 and 3.0 mg/kg bodyweight doses provided 100% lice control from Day 14 to Day 140).

For products used in long wool, ’control’ is defined as: ’Reduction of the lice population by more than 95 per cent after 90 days (or less as supported by data) in sheep examined in pen and field trials’ hence, the 1, 2 and 3 mg fluralaner/kg bodyweight has met the regulatory requirement for a long wool lice claim in this study.

| Geometric efficacy (%) | | | | | | | |
|---|---|---|---|---|---|---|---|
| Gro up | Treatment | Day 7 | Day 14 | Day 21 | Day 42 | Day 84 | Day 140 |
| 2 | Fluralaner 1.0mg/kg | 99.4% | 99.9% | 100.0% | 100.0% | 100.0% | 100.0% |
| 3 | Fluralaner 2.0mg/kg | 99.6% | 100.0% | 100.0% | 100.0% | 100.0% | 100.0% |
| 4 | Fluralaner 3.0mg/kg | 99.6% | 100.0% | 100.0% | 100.0% | 100.0% | 100.0% |

No treatment related adverse events or health problems occurred throughout the duration of the trial.

Example 2 (Reference example)

**Off-shears/Short Wool Lice Efficacy Study**

In this study the efficacy of fluralaner when administered once subcutaneously at doses of 1 and 2 mg/kg body weight within 1 week prior to shearing, within 24 hours after shearing and 6 weeks after shearing against sheep body lice *(Bovicola ovis)* infestation on Merino sheep was evaluated.

Seventy medium wool, mixed sex and age, Merino sheep with a moderate (1-5 lice per 10 cm parting) sheep body louse infestation of diazinon resistant lice were divided into seven groups of ten animals such that each group had a similar mean lice burden. Fluralaner was administered to sheep in Groups 2 and 3 seven days pre-shearing, Groups 4 and 5 within 24 hours after shearing and Groups 6 and 7 forty-two days post-shearing at either 1 mg/kg (Group 2, 4 and 6) or 2 mg/kg (Group 3, 5 and 7). Group 1 was left untreated as a negative control. All sheep were shorn on Day 0.

Lice counts were conducted on all sheep on Day -8 to -7 for allocation. Pre-treatment lice counts were conducted on Groups 2 and 3 on Day -8 to -7 (i.e. allocation counts), on Group 4 and 5 on Day -1 (pre-shearing) and on Group 6 and 7 on Day 41 (post-shearing). Post-treatment lice counts were conducted 7, 14, 21, 42, 84 and 140 days ± 1-day post-treatment. Group 1 was counted on each day that a lice count was conducted on a treated group for comparison purposes. Lice counts were performed as described in Example 1.

The percent efficacies recorded in this study are presented in the table below. The activity of the Fluralaner reached the required level (no lice detected) for the 1.0 mg/kg dose rate at all wool lengths by 21 to 84 days post-treatment. Efficacy at the 2.0 mg/kg dose rate reached the required level by 14 days post-treatment at all wool lengths except off-shears where lice persisted on 3 sheep until 21 days post-treatment and on one sheep to 84 days post-treatment.

The efficacy at both the 1 and 2 mg/kg dose rates for the sheep treated seven days before shearing, within 24 hours after shearing and 42 days after shearing was maintained out to 140 days post-treatment and no viable lice eggs were detected hence the regulatory requirement for an off-shears/short wool lice claim was met.

**Percentage Sheep Body Lice Control using the Henderson-Tilton Formula**

| Sheep Body Lice Efficacy Based on Geometric Means | | | | | | | |
|---|---|---|---|---|---|---|---|
| Group | Treatment | Day 7 | Day 14 | Day 21 | Day 41/42 | Day 84 | Day 140 |
| 2 | Fluralaner 1 mg/kg<br>Treated 7 days pre-shearing | 93.9 | 97.7 | 99.7 | 100.0 | 100.0 | 100.0 |
| 3 | Fluralaner 2 mg/kg<br>Treated 7 days pre-shearing | 96.7 | 100.0 | 100.0 | 100.0 | 100.0 | 100.0 |
| 4 | Fluralaner 1 mg/kg<br>Treated within 24 hours after shearing | 39.5 | 83.1 | 98.8 | 99.9 | 100.0 | 100.0 |
| 5 | Fluralaner 2 mg/kg<br>Treated within 24 hours after shearing | 86.6 | 97.1 | 99.2 | 99.4 | 99.9 | 100.0 |
| 6 | Fluralaner 1 mg/kg<br>Treated 42 days post-shearing | 94.4 | 99.5 | 100.0 | 100.0 | 100.0 | 100.0 |

(continued)

| Sheep Body Lice Efficacy Based on Geometric Means | | | | | | | |
|---|---|---|---|---|---|---|---|
| Group | Treatment | Day 7 | Day 14 | Day 21 | Day 41/42 | Day 84 | Day 140 |
| 7 | Fluralaner 2 mg/kg<br>Treated 42 days post-shearing | 96.8 | 100.0 | 100.0 | 100.0 | 100.0 | 100.0 |

Example 3

**Long Wool Lice Efficacy Study**

In this study the efficacy of fluralaner when administered once subcutaneously at doses of 1 and 2 mg/kg body weight against sheep body lice *(Bovicola ovis)* infestation on Merino sheep with 3 months wool growth was evaluated.

Thirty medium wool Merino ewes with a moderate (1-5 lice per 10 cm parting) sheep body lice infestation of diazinon resistant lice were divided into three groups of ten animals such that each group had a similar mean lice burden. Fluralaner was administered to sheep in Groups 2 and 3 on Day 0 at 1 and 2 mg fluralaner/kg bodyweight respectively. Group 1 was left untreated as a negative control.

Pre-treatment lice counts were conducted on Day -1 and used to allocate sheep to treatment groups. Post-treatment lice counts were conducted 7, 14, 21, 42, 84 and 140 days ± 1-day post-treatment. Lice counts were performed as discussed in Example 1.

The percent efficacies recorded in this study are presented in the table below. Activity for the 1.0 mg/kg dose rate by 42 days post-treatment. Efficacy at the 2.0 mg/kg dose rate reached the required level by 14 days post-treatment. The efficacy at both the 1 and 2 mg/kg dose rates was maintained out to 140 days post-treatment hence the regulatory requirement for a long- wool lice claim for sheep with 3 months wool was met and exceeded.

**Percentage Sheep Body Lice Control using the Henderson-Tilton Formula**

| Sheep Body Lice Efficacy Based on Geometric Means | | | | | | | |
|---|---|---|---|---|---|---|---|
| Group | Treatment | Day 7 | Day 14 | Day 21 | Day 42 | Day 84 | Day 140 |
| 2 | Fluralaner 1 mg/kg - 3 months wool | 81.8 | 97.2 | 99.6 | 100.0 | 100.0 | 100.0 |
| 3 | Fluralaner 2 mg/kg - 3 months wool | 95.2 | 100.0 | 100.0 | 100.0 | 100.0 | 100.0 |

Example 4 (Reference example)

**Sucking and biting lice efficacy study in cattle**

This study determined the efficacy (via reductions in total lice counts) of fluralaner in an injectable suspension formulation against natural infestations of cattle sucking (*Haematopinus eurysternus, Linognathus vituli*) and biting/ chewing lice (*Bovicola bovis*) when applied at a single dose to young, lice infested cattle. Treatment efficacy was determined by whole body lice counts post treatment and comparison of treated and untreated (placebo) group mean lice counts.

The naturally acquired louse infestations consisted of 2 species of sucking lice (*Haematopinus eurysternus* and *Linognathus vituli*) and the biting louse *Bovicola bovis.* Each animal was examined in a cattle crush prior to being selected from the parent mob to ensure that more than 30 sucking lice (combined total of both sucking lice species) were present.

Fluralaner at a dose level of 1.0 mg/ kg bw and Control Product were administered to cattle by subcutaneous injection..

| Group | IVP & CP Details | Dose Level | Route | Treatment Day (s) | Number of Animals |
|---|---|---|---|---|---|
| 1 | Controls -Placebo Sterile saline solution | N/A | Subcutaneous Injection | 0 | 7 |
| 2 | Fluralaner Injectable | 1.0 mg/kg | Subcutaneous Injection | 0 | 7 |

The numbers of live adults and live nymphs for each species of lice on each animal were counted at 7-day intervals starting with pre-treatment counts on Day -1 and post-treatment on Days 7, 14, 21, 28, 35, 42, 49 and 56.

Fluralaner administered to cattle at 1 mg/kg demonstrated high efficacy against the biting and sucking cattle lice species during the study. The IVP reached 97.69% and 99.89% efficacy (based on Arithmetic Means) on Days 7 and 14 respectively against the sucking lice. The efficacy against the biting lice *B. bovis* was reached on Day 7 (91.74% efficacy), however this increased to 100% by Day 14.

There were no lice of any species found on any of the treated cattle from Day 21 until the study conclusion on Day 56.

Example 5

**Persistency Determination**

In this study the persistent efficacy of fluralaner when administered once subcutaneously at doses of 1.0, 2.0 and 3.0 mg/kg body weight against sheep body lice *(Bovicola ovis)* infestation on Merino sheep with 5 months wool growth was evaluated.

Seventy-two (72) lice free Merino sheep were divided into twelve groups of six animals such that each group had a similar mean body weight. Fluralaner was administered to sheep in three groups each at 1.0, 2.0 and 3.0 mg fluralaner/kg bodyweight. The dose was administered subcutaneously. Three groups were left untreated as negative control groups.

From Day 21-28, 49-56 and Day 77-84 post-treatment one group of untreated negative control sheep and one group of sheep treated at 1.0, 2.0 and 3.0 mg/kg were challenged with lice by close confinement with lice infested sheep and manual transfer of lice. Following the challenges lice counts were conducted 7, 14, 21, 42 and 84 days ± 1-day post-challenge (not all counts conducted for all challenges).

No lice or viable lice eggs were found on the sheep treated at the 3.0 mg/kg dose rate after the Day 21-28 challenge 7, 14, 21, 42 and 84 days ± 1-day post-challenge.

## Claims

1. An isoxazoline compound of Formula (I)

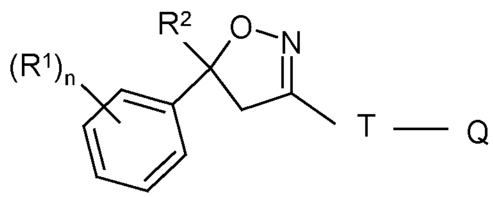

Formula (I),

wherein

$R^1$ is halogen, $CF_3$, $OCF_3$, CN,
n is an integer from 0 up to and including 3, preferably 1, 2 or 3,
$R^2$ is $C_1$-$C_3$-haloalkyl, preferably $CF_3$ or $CF_2Cl$,
T is a 5 to 12 membered mono or bicyclic ring system, which is optionally substituted by one or more radicals Y,
Y is methyl, halomethyl, halogen, CN, $NO_2$, $NH_2$-C=S, or two adjacent radicals Y form together a chain, especially a three or four-membered chain;
Q is X-$NR^3R^4$, $NR^5$-$NR^6$-X-$R^3$, X-R3 or a 5-membered N-heteroaryl ring, which is optionally substituted by one

or more radicals;

X is $CH_2$, $CH(CH_3)$, CH(CN), CO, CS,

$R^3$ is hydrogen, methyl, haloethyl, halopropyl, halobutyl, methoxymethyl, methoxyethyl, halomethoxymethyl, ethoxymethyl, haloethoxymethyl, propoxymethyl, ethylaminocarbonylmethyl, ethylaminocarbonylethyl, dimethoxyethyl, propynylaminocarbonylmethyl, N-phenyl-N-methyl-amino, haloethylaminocarbonylmethyl, haloethylaminocarbonylethyl, tetrahydrofuryl, methylaminocarbonylmethyl, (N,N-dimethylamino)-carbonylmethyl, propylaminocarbonylmethyl, cyclopropylaminocarbonylmethyl, propenylaminocarbonylmethyl, halo-ethylaminocarbonylcyclopropyl, alkylsulfanyl, alkylsufinalkyl, alkylsulfonalkyl, cycloalkyl

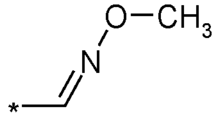 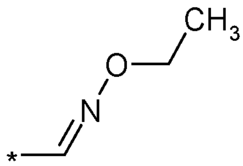 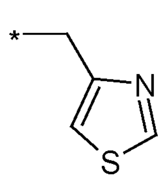

$R^3$-1 $R^3$-2 $R^3$-3

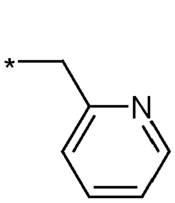 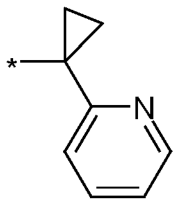 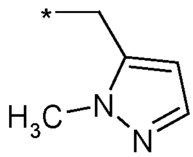 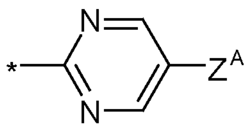

i. $R^3$-4 $R^3$-5 $R^3$-6 $R^3$-7

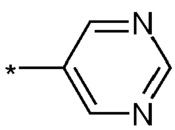 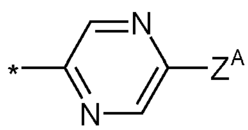 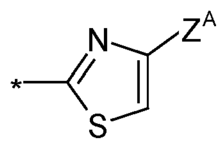

ii. $R^3$-8 $R^3$-9 $R^3$-10

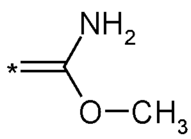 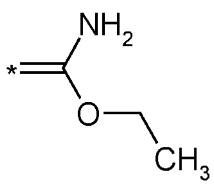 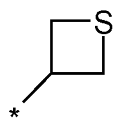 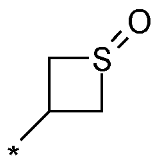

$R^3$-11 $R^3$-12 $R^3$-13 $R^3$-14

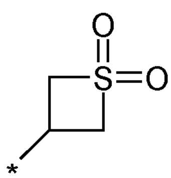 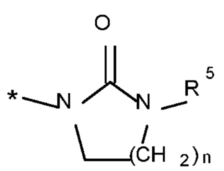 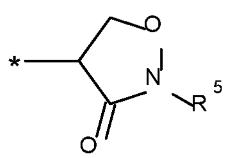 or 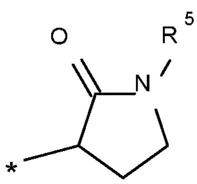

iii. $R^3$-15 $R^3$-16 $R^3$-17 $R^3$-18

wherein $Z^A$ is hydrogen, halogen, cyano, halomethyl, preferably $CF_3$;

$R^4$ is hydrogen, ethyl, methoxymethyl, halomethoxymethyl, ethoxymethyl, haloethoxymethyl, propoxymethyl, methylcarbonyl, ethylcarbonyl, propylcarbonyl, cyclopropylcarbonyl, methoxycarbonyl, methoxymethylcarbonyl, aminocarbonyl, ethylaminocarbonylmethyl, ethylaminocarbonylethyl, dimethoxyethyl, propynylaminocarbonylmethyl, haloethylaminocarbonylmethyl, cyanomethylaminocarbonylmethyl, or haloethylaminocarbonylethyl;
$R^5$ is hydrogen, alkyl or haloalkyl;
$R^6$ is hydrogen, alkyl or haloalkyl;
or $R^3$ and $R^4$ together form a substituent selected from the group consisting of:

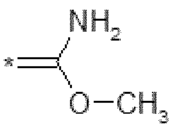,
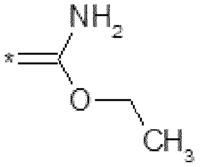,
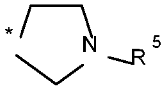,
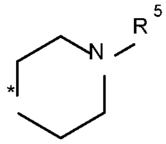,
and
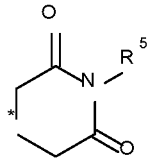

or a salt or solvate thereof for use in the treatment and/or protection of sheep from *Bovicola ovis* chewing lice infestation after a single subcutaneous administration of a composition comprising an effective amount of at least one isoxazoline compound of Formula (I), wherein the composition is administered as solution or suspension to sheep once > 6 weeks after shearing

**2.** The compound for use according to claim 1 wherein the isoxazoline compound of formula (I) is fluralaner.

**3.** The compound for use according to any one of claims 1 to 2 wherein the effective amount of the isoxazoline compound is between 1 and 3 mg/ kg bodyweight of the animal.

**4.** The compound for use according to any one of claims 1 to 3 wherein the composition is administered once to a sheep mob with at least one animal diagnosed with chewing lice infestation.

**5.** The compound for use according to any one of claims 1 to 4 wherein the effective amount of the isoxazoline compound is sufficient to eliminate an existing chewing lice infestation within 15 days after administration.

**6.** The compound for use according to any one of claims 1 to 5 wherein the effective amount of the isoxazoline compound is sufficient after a single subcutaneous administration to eliminate all live lice from treated sheep when investigated 140 days after treatment.

**7.** The compound for use according to any one of claims 1 to 6 wherein the effective amount of the isoxazoline compound is sufficient after a single subcutaneous administration to protect sheep for at least 2 weeks from chewing lice infestation.

**8.** The compound for use according to any one of claims 1 to 7 wherein the chewing lice are resistant to any one of organophosphates, synthetic pyrethroids, neonicotinoids, spinosyn or benzoyl phenyl urea compounds.

**9.** The compound for use according to any one of claims 1 to 8 wherein the composition comprises another active pharmaceutical ingredient.

**10.** The compound for use according to claim 9 wherein the composition comprises at least one endoparasiticide.

**Patentansprüche**

**1.** Isoxazolinverbindung der Formel (I)

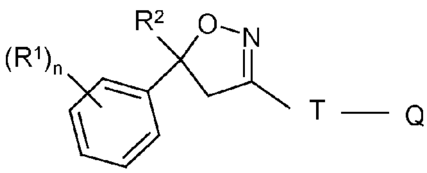

Formel (I),

wobei

$R^1$ für Halogen, $CF_3$, $OCF_3$, CN steht,
n für eine ganze Zahl von 0 bis einschließlich 3, vorzugsweise 1, 2 oder 3, steht,
$R^2$ für $C_1$-$C_3$-Halogenalkyl, vorzugsweise $CF_3$ oder $CF_2Cl$, steht,
T für ein 5- bis 12-gliedriges mono- oder bicyclisches Ringsystem steht, das gegebenenfalls durch einen oder mehrere Reste Y substituiert ist,
Y für Methyl, Halogenmethyl, Halogen, CN, $NO_2$, $NH_2$-C=S steht oder zwei benachbarte Reste Y zusammen eine Kette, insbesondere eine drei- bis viergliedrige Kette, bilden, Q für X-$NR^3R^4$, $NR^5$-$NR^6$-X-$R^3$, X-$R^3$ oder einen 5-gliedrigen N-Heteroarylring steht, der gegebenenfalls durch einen oder mehrere Reste substituiert ist,
X für $CH_2$, $CH(CH_3)$, CH(CN), CO, CS steht,
$R^3$ für Wasserstoff, Methyl, Halogenethyl, Halogenpropyl, Halogenbutyl, Methoxymethyl, Methoxyethyl, Halogenmethoxymethyl, Ethoxymethyl, Halogenethoxymethyl, Propoxymethyl, Ethylaminocarbonylmethyl, Ethylaminocarbonylethyl, Dimethoxyethyl, Propinylaminocarbonylmethyl, N-Phenyl-N-methylamino, Halogenethylaminocarbonylmethyl, Halogenethylaminocarbonylethyl, Tetrahydrofuryl, Methylaminocarbonylmethyl, (N,N-Dimethyl-amino)carbonylmethyl, Propylaminocarbonylmethyl, Cyclopropylaminocarbonylmethyl, Propenylaminocarbonylmethyl, Halogenethylaminocarbonylcyclopropyl, Alkylsulfanyl, Alkylsufinalkyl, Alkylsulfonalkyl, Cycloalkyl,

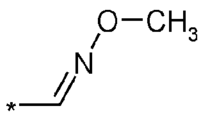 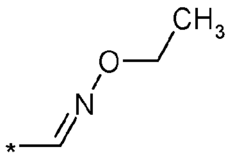 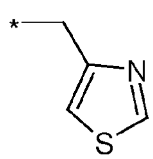

**$R^3$-1** **$R^3$-2** **$R^3$-3**

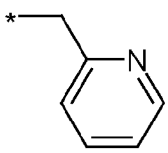 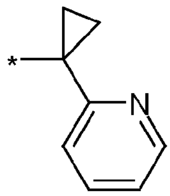 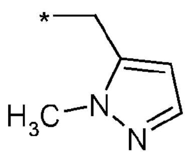 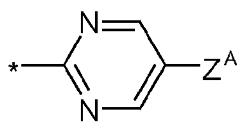

i. **$R^3$-4** **$R^3$-5** **$R^3$-6** **$R^3$-7**

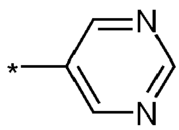 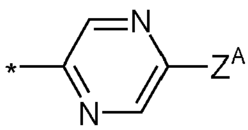 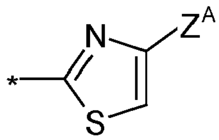

ii. **$R^3$-8** **$R^3$-9** **$R^3$-10**

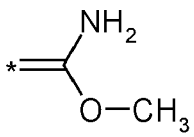 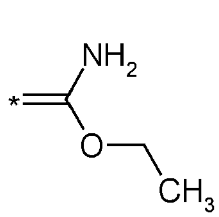 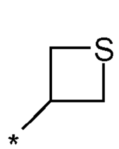 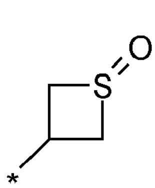

**$R^3$-11** **$R^3$-12** **$R^3$-13** **$R^3$-14**

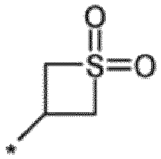 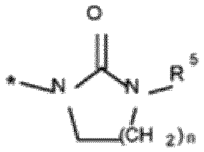 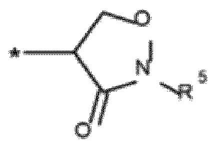 of 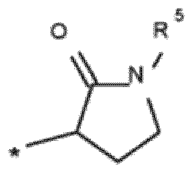

iii. R³-15 R³-16 R³-17 R³-18

steht, wobei $Z^A$ für Wasserstoff, Halogen, Cyano, Halogenmethyl, vorzugsweise $CF_3$, steht,

$R^4$ für Wasserstoff, Ethyl, Methoxymethyl, Halogenmethoxymethyl, Ethoxymethyl, Halogenethoxymethyl, Propoxymethyl, Methylcarbonyl, Ethylcarbonyl, Propylcarbonyl, Cyclopropylcarbonyl, Methoxycarbonyl, Methoxymethylcarbonyl, Aminocarbonyl, Ethylaminocarbonylmethyl, Ethylaminocarbonylethyl, Dimethoxyethyl, Propinylaminocarbonylmethyl, Halogenethylaminocarbonylmethyl, Cyanomethylaminocarbonylmethyl, oder Halogenethylaminocarbonylethyl steht,

$R^5$ für Wasserstoff, Alkyl oder Halogenalkyl steht,

$R^6$ für Wasserstoff, Alkyl oder Halogenalkyl steht, oder $R^3$ und $R^4$ zusammen einen Substituenten ausgewählt aus der folgenden Gruppe bilden:

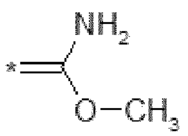, 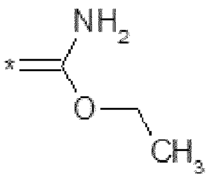, 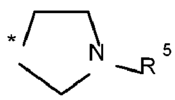, 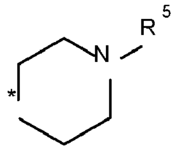

und

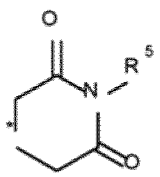

oder ein Salz oder Solvat davon zur Verwendung bei der Behandlung und/oder dem Schutz von Schafen gegen Befall durch *Bovicola* ovis-Haarlinge nach einer einzelnen subkutanen Verabreichung einer eine wirksame Menge mindestens einer Isoxazolinverbindung der Formel (I) umfassenden Zusammensetzung, wobei die Zusammensetzung den Schafen einmal > 6 Wochen nach dem Scheren als Lösung oder Suspension verabreicht wird.

2. Verbindung zur Verwendung nach Anspruch 1, wobei es sich bei der Isoxazolinverbindung der Formel (I) um Fluralaner handelt.

3. Verbindung zur Verwendung nach Anspruch 1 oder 2, wobei die wirksame Menge der Isoxazolinverbindung 1 bis 3 mg/kg Körpergewicht des Tieres beträgt.

4. Verbindung zur Verwendung nach einem der Ansprüche 1 bis 3, wobei die Zusammensetzung einmalig an eine Schafherde verabreicht wird, in der bei mindestens einem Tier ein Haarlingsbefall diagnostiziert wurde.

5. Verbindung zur Verwendung nach einem der Ansprüche 1 bis 4, wobei die wirksame Menge der Isoxazolinverbindung ausreicht, um einen vorliegenden Haarlingsbefall innerhalb von 15 Tagen nach der Verabreichung zu eliminieren.

6. Verbindung zur Verwendung nach einem der Ansprüche 1 bis 5, wobei die wirksame Menge der Isoxazolinverbindung ausreicht, um nach einer einzelnen subkutanen Verabreichung bei einer Untersuchung 140 Tage nach der Behandlung alle lebenden Haarlinge von behandelten Schafen zu eliminiert zu haben.

**7.** Verbindung zur Verwendung nach einem der Ansprüche 1 bis 6, wobei die wirksame Menge der Isoxazolinverbindung ausreicht, um nach einer einzelnen subkutanen Verabreichung Schafe mindestens 2 Wochen gegen Haarlingsbefall zu schützen.

**8.** Verbindung zur Verwendung nach einem der Ansprüche 1 bis 7, wobei die Haarlinge resistent gegen Organophosphate, synthetische Pyrethroide, Neonicotinoide, Spinosyn oder Benzoylphenylharnstoffverbindungen sind.

**9.** Verbindung zur Verwendung nach einem der Ansprüche 1 bis 8, wobei die Zusammensetzung einen weiteren pharmazeutischen Wirkstoff umfasst.

**10.** Verbindung zur Verwendung nach Anspruch 9, wobei die Zusammensetzung mindestens ein Endoparasitizid umfasst.

## Revendications

**1.** Composé de type isoxazoline de formule (I)

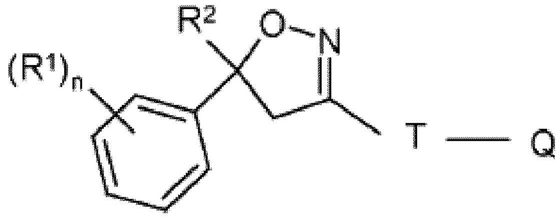

formule (I),

$R^1$ étant halogène, $CF_3$, $OCF_3$, CN,
n étant un entier de 0 jusqu'à 3 inclus, préférablement 1, 2 ou 3,
$R^2$ étant $C_1$-$C_3$-halogénoalkyle, préférablement $CF_3$ ou $CF_2Cl$,
T étant un système cyclique monocyclique ou bicyclique à 5 à 12 chaînons, qui est éventuellement substitué par un ou plusieurs radicaux Y,
Y étant méthyle, halogénométhyle, halogène, CN, $NO_2$, $NH_2$-C=S, ou deux radicaux Y adjacents formant ensemble une chaîne, notamment une chaîne à trois ou quatre chaînons ;
Q étant X-$NR^3R^4$, $NR^5$-$NR^6$-X-$R^3$, X-R3 ou un cycle N-hétéroaryle à 5 chaînons, qui est éventuellement substitué par un ou plusieurs radicaux ;
X étant $CH_2$, $CH(CH_3)$, CH(CN), CO, CS,
$R^3$ étant hydrogène, méthyle, halogénoéthyle, halogénopropyle, halogénobutyle, méthoxyméthyle, méthoxyéthyle, halogénométhoxyméthyle, éthoxyméthyle, halogénoéthoxyméthyle, propoxyméthyle, éthylaminocarbonylméthyle, éthylaminocarbonyléthyle, diméthoxyéthyle, propynylaminocarbonylméthyle, N-phényl-N-méthylamino, halogénoéthylaminocarbonylméthyle, halogénoéthylaminocarbonyléthyle, tétrahydrofuryle, méthylaminocarbonylméthyle, (N,N-diméthylamino)-carbonylméthyle, propylamino-carbonylméthyle, cyclopropylaminocarbonylméthyle, propénylaminocarbonylméthyle, halogéno-éthylaminocarbonylcyclopropyle, alkylsulfanyle, alkylsufinalkyle, alkylsulfonalkyle, cycloalkyle

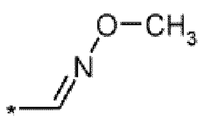

**$R^3$-1**

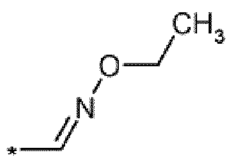

**$R^3$-2**

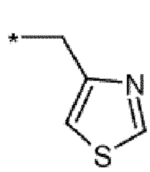

**$R^3$-3**

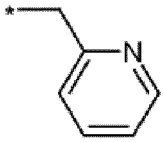 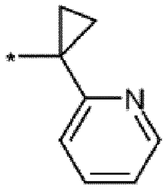 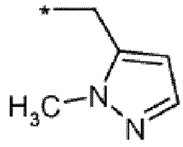 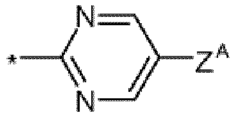

i. **R³-4** **R³-5** **R³-6** **R³-7**

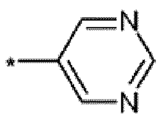 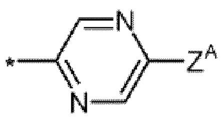 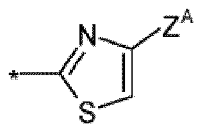

ii. **R³-8** **R³-9** **R³-10**

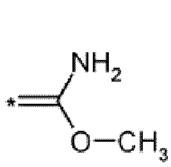 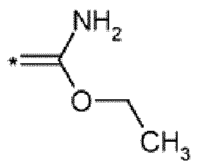 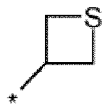 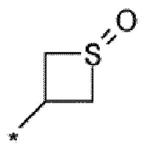

**R³-11** **R³-12** **R³-13** **R³-14**

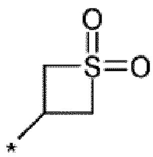 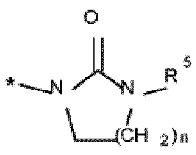 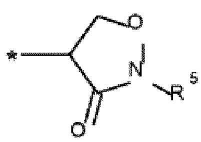 ou 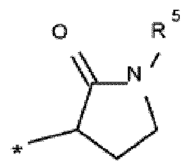

iii. **R³-15** **R³-16** **R³-17** **R³-18**

$Z^A$ étant hydrogène, halogène, cyano, halogénométhyle, préférablement $CF_3$ ;
$R^4$ étant hydrogène, éthyle, méthoxyméthyle, halogénométhoxyméthyle, éthoxyméthyle, halogénoéthoxyméthyle, propoxyméthyle, méthylcarbonyle, éthylcarbonyle, propylcarbonyle, cyclopropylcarbonyle, méthoxycarbonyle, méthoxyméthylcarbonyle, aminocarbonyle, éthylaminocarbonylméthyle, éthylaminocarbonyléthyle, diméthoxyéthyle, propynylaminocarbonylméthyle, halogénoéthylaminocarbonylméthyle, cyanométhylaminocarbonylméthyle, ou halogénoéthylaminocarbonyléthyle ;
$R^5$ étant hydrogène, alkyle ou halogénoalkyle ;
$R^6$ étant hydrogène, alkyle ou halogénoalkyle ;
ou $R^3$ et $R^4$ formant ensemble un substituant choisi dans le groupe constitué par :

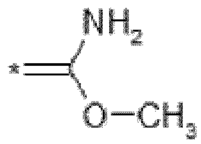, 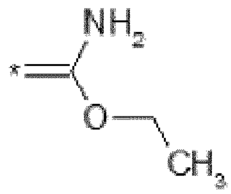, 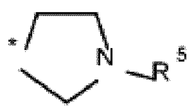, 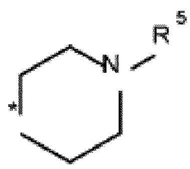

et

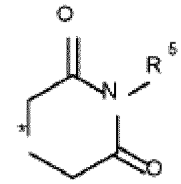

ou sel ou solvate correspondant pour une utilisation dans le traitement et/ou la protection de moutons contre une infestation par des poux broyeurs *Bovicola ovis* après une seule administration sous-cutanée d'une composition comprenant une quantité efficace d'au moins un composé de type isoxazoline de formule (I), la composition étant administrée en tant que solution ou suspension aux moutons une fois > 6 semaines après la tonte.

**2.** Composé pour une utilisation selon la revendication 1, le composé de type isoxazoline de formule (I) étant le fluralaner.

**3.** Composé pour une utilisation selon l'une quelconque des revendications 1 à 2, la quantité efficace du composé de type isoxazoline étant comprise entre 1 et 3 mg/kg de poids corporel de l'animal.

**4.** Composé pour une utilisation selon l'une quelconque des revendications 1 à 3, la composition étant administrée une fois à un troupeau de mouton comportant au moins un animal diagnostiqué d'une infestation par des poux broyeurs.

**5.** Composé pour une utilisation selon l'une quelconque des revendications 1 à 4, la quantité efficace du composé de type isoxazoline étant suffisante pour éliminer une infestation par des poux broyeurs existante dans les 15 jours après une administration.

**6.** Composé pour une utilisation selon l'une quelconque des revendications 1 à 5, la quantité efficace du composé de type isoxazoline étant suffisante après une unique administration sous-cutanée pour éliminer tous les poux vivants de moutons traités lors d'une investigation 140 jours après traitement.

**7.** Composé pour une utilisation selon l'une quelconque des revendications 1 à 6, la quantité efficace du composé de type isoxazoline étant suffisante après une unique administration sous-cutanée pour protéger des moutons pendant au moins 2 semaines d'une infestation par des poux broyeurs.

**8.** Composé pour une utilisation selon l'une quelconque des revendications 1 à 7, les poux broyeurs étant résistants à l'un quelconque parmi des organophosphates, des pyréthroïdes synthétiques, des néonicotinoïdes, une spinosyne ou des composés de type benzoylphénylurée.

**9.** Composé pour une utilisation selon l'une quelconque des revendications 1 à 8, la composition comprenant un autre ingrédient pharmaceutique actif.

**10.** Composé pour une utilisation selon la revendication 9, la composition comprenant au moins un endoparasiticide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013184006 A
- WO 2014051440 A
- WO 2019115492 A
- US 20070066617 A
- WO 2005085216 A
- WO 2007079162 A
- WO 2009002809 A
- WO 2009024541 A
- WO 2009003075 A
- WO 2010070068 A
- WO 2010079077 A
- WO 20090080250 A
- WO 2009080250 A
- WO 2011075591 A
- WO 2011124998 A


### Non-patent literature cited in the description


- **HOLDSWORTH, PA et al.** World Association for the Advancement of Veterinary Parasitology guidelines for evaluating the efficacy of ectoparasiticides against biting lice, sucking lice and sheep keds on ruminants. *Veterinary Parasitology,* 2006, vol. 136, 45-54
- *CHEMICAL ABSTRACTS,* 1398609-39-6
- *CHEMICAL ABSTRACTS,* 1369852-71-0
- *CHEMICAL ABSTRACTS,* 1621436
- *CHEMICAL ABSTRACTS,* 928789-76-8
- *CHEMICAL ABSTRACTS,* 1164267-94-0
- *CHEMICAL ABSTRACTS,* 1231754-09-8
- **MARILYN N. MARTINEZ ; DANIELLE LINDQUIST ; SANJA MODRIC.** Terminology Challenges: Defining Modified Release Dosage Forms in Veterinary Medicine. *Journal of Pharmaceutical Sciences,* August 2010, vol. 99 (8
- *Gennaro, Remington: The Science and Practice of Pharmacy,* 2000